# EUROPEAN PATENT APPLICATION

(11) **EP 2 653 166 A2**
(43) Date of publication of application: **23.10.2013**
(21) Application number: 12007936.3
(22) Date of filing: 30.07.2008
(51) Int. Cl.: A61K 38/28, A61K 38/08, A61K 38/17, A61K 31/553, A61K 31/403, A61K 31/404, A61K 31/7088, A61P 17/02

(54) **Pharmaceutical composition and related methods**

(30) Priority: 30.07.2007 US 962706 P
(62) Divisional of application: 08789725.2
(71) Applicant: Healor Ltd., 74140 Ness-Ziona (IL)
(72) Inventor: Tennenbaum, Tamar, 96105 Jerusalem (IL); Braiman-Wiksman, Liora, 75400 Rishon Lezion (IL); Solomonik, Inessa, Ganey Tikva 55900 (IL); Levy-Hacham, Ofra, Ness Ziona 74113 (IL); Brener, Ephraim, 75400 Rishon Lezion (IL)
(74) Representative: Helbig, Christian

(57) **Abstract**

The present invention relates to compositions and methods for accelerating the healing process of wounds wherein the compounds are free of Mg2+ and Mg2+ cations.

## Description

### PRIORITY

This application claims priority to U.S. Provisional Patent Application No. 60/962,706 filed July 30, 2007 and entitled "Pharmaceutical Composition" the entire contents of which are herein incorporated by reference.

### FIELD OF THE DISCLOSURE

The present disclosure relates to compositions and methods for accelerating the healing of wounds, increasing the closure of skin wounds, and decreasing inflammation at the site of a skin wound.

### BACKGROUND

Skin is a complex tissue structured as distinct layers, namely, the epidermis, dermis and hypodermis, each possessing a different cell characterization and physiological significance (Fuchs and Byrne 1994; Goldsmith 1991).

The epidermis is stratified squamous epithelium in which cells undergoing growth and differentiation are strictly compartmentalized (Fuchs and Byrne 1994). In a normal physiological state, proliferation is confined to the basal cells that adhere to the basement membrane. Differentiation is a spatial process in which basal cells lose their adhesion to the basement membrane, cease DNA synthesis and undergo a series of morphological and biochemical changes. The ultimate maturation step is the production of the cornified layer forming the protective barrier of the skin (Tennenbaum *et al.* 1991; Wysocki 1999).

The dermis is mainly composed of matrix fibers and contains various cell types. In addition, all skin appendages, namely, microvasculature, sweat and sebaceous glands, sensory nerves and hair follicles, are localized in the dermis. The dermis has been attributed the supporting role of skin nourishment, maintaining the epidermis and the route by which signals from other parts of the body reach the outer layer (Green 1977; Wysocki 1999). The hypodermis is the deepest layer of the skin, mainly consisting of adipose cells, also known as the subcutaneous fat layer. Until recently, this layer has been thought to have the role of insulation from the external temperature changes and mechanical support to the upper layers of the skin (Nash *et al.* 2004; Querleux *et al.* 2002).

In skin, the continued renewal of the stratified epidermis is maintained by a sequential and highly specialized process leading to the production of the non-viable, cornified squames, which together with lipids derived from secreted lamellar bodies constitutes a protective water barrier of the body. Proliferating basal cells adhere to an epidermis-specific basement membrane. The keratinocyte differentiation process is closely linked to the loss of cell contact with the basement membrane; as basal cells migrate into the more superficial spinous layer they lose their proliferative capability. Further maturation to the granular cell compartment is followed by, formation of the rigid cornified envelopes is associated with autolysis of intracellular organelles and programmed cell death, giving rise to the mature squames (Adams and Watt 1990; Eckert 1989; Yuspa *et al.* 1980).

Open cutaneous wounds routinely heal by a process which comprises six major components: (i) inflammation; (ii) fibroblast proliferation; (iii) blood vessel proliferation; (iv) connective tissue synthesis; (v) epithelialization; and (vi) wound contraction. Wound healing is impaired when these components, either individually or as a whole, do not function properly. Numerous factors can affect wound healing, including malnutrition, infection, pharmacological agents (*e.g*., actinomycin and steroids), advanced age and diabetes (Keast and Orsted 1998; Kirsner and Eaglstein 1993; Williams and Armstrong 1998).

Diabetes mellitus, a common form of diabetes, is characterized by impaired insulin signaling, elevated plasma glucose and a predisposition to develop chronic complications involving several distinctive tissues. Among all the chronic complications of diabetes mellitus, impaired wound healing leading to foot ulceration is among the least well studied (Goodson and Hunt 1979; Grunfeld 1992). Yet skin ulceration in diabetic patients takes a staggering personal and financial cost. Moreover, foot ulcers and the subsequent amputation of a lower extremity are the most common causes of hospitalization among diabetic patients. In diabetes, the wound healing process is impaired and healed wounds are characterized by diminished wound strength (Shaw and Boulton 1997). The defect in tissue repair has been related to several factors including neuropathy, vascular disease and infection (Mousley 2003; Silhi 1998). However, additional mechanisms whereby the diabetic state associated with abnormal insulin signaling impairs wound healing and alters the physiology of skin have not been elucidated. There is also a common problem of wound healing following surgical procedures in various parts of the body that is influenced by age and development of chronic diseases such as diabetes and obesity. In surgical settings, a third of the patients suffer from a delay in wound healing attributed to their physiological state as well as the development of associated infections at the wound site (Diegelmann and Evans 2004).

Skin wounds are commonly found in animals including horses, dogs, cats and live stock. In animals wounds have a variety of common disease presentations that require wound management. Therefore veterinary dermatology is one of the most rapidly growing disciplines in veterinary medicine.

Generally, many of these wounds heal by second-intention. This process takes a long time, especially when the limbs are involved. In animals, as well as in humans, the wound healing process can be complicated by factors such as contamination, infection or dehiscence, that are often the cause of prolonged healing times or inappropriate wound closure (Grunfeld 1992; Knol and Wisselink 1996; Yeruham *et al.* 1992; Yim *et al.* 2007).

Typically, wound healing requires induction (activation) of the formation of new epidermis and granulation tissue and a reduction in inflammation. These processes are also essential in animals for the healing of various acute and chronic wounds such as post-surgical wounds, acral lick ulcers, diabetic ulcers and more. Horses suffer from chronic wounds (e.g. "Proud flesh") that are caused by overabundance of granulation tissue in which proliferation of fibroblasts and angiogenesis are pathologically increased. This abnormal granulation tissue overgrows above the level of the epithelium and physically blocks the access of adjacent skin that otherwise might grow over the area. The mechanism of this uncontrolled growth of fibroblasts is unknown. The only treatment available involves surgical removal of over-abandoned tissue, pressure bandaging and corticosteroids. The treatment takes a prolonged time (from 5-8 months) and the lesions are usually recurrent (De, I and Theoret 2004; Stone 1986).

Other specific pathologies in animals include Acral Lick Dermatitis and rodent ulcers in dogs. Acral lick dermatitis is a common problem in dogs which refers to the raised reddened, tough, rubbery tissue associated with dog lesions which result from repetitive licking of the same area. Despite numerous strategies in the treatment of acral lick dermatitis, healing rates and efficacy are insufficient and in many cases recurrence of the ulcer occurs (White 1990; Yeruham *et al.* 1992).

Protein kinase C (PKC) is a family of phospholipid dependent enzymes that catalyze the covalent transfer of phosphate from ATP to serine and threonine residues on proteins, and which plays an important role in regulating skin physiology. Phosphorylation of the substrate proteins induces a conformational change resulting in modification of their functional properties. So far, 11 isoforms were found to be involved in a variety of cellular functions and signal transduction pathways regulating proliferation, differentiation, cell survival, and death (Nishizuka 1995). The specific cofactor requirements, tissue localization and cellular compartmentalization suggest differential functions and fine tuning of specific signaling cascades for each isoform. Thus, specific stimuli can lead to differential responses via isoform specific PKC signaling regulated by their expression, localization and phosphorylation status in particular biological settings. PKC isoforms are activated by a variety of extra cellular signals and, in turn, modify the activities of cellular proteins including receptors, enzymes, cytoskeletal proteins and transcription factors. Accordingly, the PKC family plays a central role in cellular signal processing.

A prototype of the protein kinase C (PKC) family of serine/ threonine kinases was first described by Nishizuka and co workers (Kikkawa *et al.* 1989), who initially discovered a PKC that is activated by diacylglycerol (DAG) which is a degradation product of phosphatidylinositol (Castagna *et al.* 1982). Other studies revealed that PKC is the intracellular receptor of tumor promoting phorbol esters.

All PKC family members share a structural backbone, which can be divided into two major domains: a regulatory domain at the N-terminus, and a catalytic domain at the C-terminus. The regions are categorized as conserved regions (C1-C4) and regions that vary between isoforms (V1-V5) (Nishizuka 1988), *supra.* In addition, PKCs exhibit a pseudosubstrate domain in the regulatory region, closely resembling the substrate recognition motif, which blocks the recognition site and prevents activation (Blumberg 1991; House and Kemp 1987). The PKC family of isoforms can be divided into 3 major groups based on their structural characteristics and cofactor requirements. These include the classical cPKC (α, βI, βII, and y), novel nPKC (δ, ε, η, 0), and the atypical aPKC (ζ and 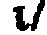 /λ) isoforms (Azzi *et al.* 1992; Kikkawa *et al.* 1989; Svetek *et al.* 1995).

All PKC isoforms require components of the phospholipid bilayer, for their activation. Classical cPKCs are calcium (Ca⁺²) dependent and also require DAG or DAG analogs such as phorbol esters for activation. The novel nPKCs are independent of Ca²⁺ but still require DAG or phorbol esters for maximal activation (Kazanietz *et al.* 1993). The atypical, aPKCs, are independent of Ca²⁺ and do not require DAG or phorbol esters but require phosphatidylserine for activation (Chauhan *et al.* 1990). In addition, a major component of substrate recognition is the pseudosubstrate region within the regulatory domain which controls the regulatory mechanisms implicated in specific activities of PKC isoforms in cellular signaling and is associated with phosphorylation of distinct target substrates (Eichholtz *et al.* 1993; Hofmann 1997).

Five PKC isoforms - α, δ, ε, η and ζ - have been identified in skin epidermis *in vivo* and in cultured keratinocytes. However, other PKC isoforms such as β and γ were detected in the dermal layer of skin. Furthermore, the type of PKC isoform and pattern of PKC distribution vary among different tissues and may also change as a function of phenotype. Importantly, PKC isoforms are distributed in both basal and differentiating skin keratinocytes *in vivo* and *in vitro* and may play a role in the wound healing.

Thus, there is a need for improved compositions and methods that modulate PKC activity to help treat skin wounds and other chronic wounds.

### SUMMARY OF THE DISCLOSURE

The disclosure generally relates to pharmaceutical compositions that contain bioactive skin wound healing and or anti-inflammatory agents that are free of calcium and magnesium ions, and to methods of treating skin wounds and/or inflammation with the pharmaceutical compositions. Preferably the pharmaceutical compositions are suitable for topical or local administration, especially subcutaneous administration.

One aspect of the disclosure is a composition comprising a delta-PKC activator, an alpha-PKC inhibitor, and a pharmaceutically acceptable carrier that is free of Ca²⁺ and Mg²⁺ cations.

Another aspect of the disclosure is a composition comprising an insulin, a peptide consisting of the amino acid sequence shown in SEQ ID NO: 1 which has a myristoylated amino acid residue at its amino terminus, and an aqueous pharmaceutically acceptable carrier comprising 0.2 g/L KCI, 0.2 g/L anhydrous KH₂PO₄, 8 g/L NaCl, and 1.15 g/L anhydrous Na₂HPO₄ that is free of Ca²⁺ and Mg²⁺ cations.

Preferably the pharmaceutically acceptable carrier includes phosphate or phosphate-containing compounds suitable for buffering the composition. A particularly preferred embodiment includes 0.2 L KCI, 0.2 g/L anhydrous KH₂PO₄, 8 g/L NaCl and 1.15 g/L anhydrous Na₂HPO₄. Such pharmaceutically acceptable carriers are also an aspect of the present invention, and can be prepared by admixing the required ingredients to provide the pharmaceutically acceptable carrier that does not contain calcium or magnesium ions.

Another aspect of the disclosure is a composition comprising a delta-PKC activator, an alpha-PKC inhibitor, a pharmaceutically acceptable carrier that is free of Ca²⁺ and Mg²⁺ cations, and a drug eluting scaffold.

Another aspect of the disclosure is a pharmaceutical composition produced by a process comprising the steps of providing a delta-PKC activator, an alpha-PKC inhibitor, and a pharmaceutically acceptable carrier that is free of Ca²⁺ and Mg²⁺ cations; and combining the delta-PKC activator, alpha-PKC inhibitor, and the pharmaceutically acceptable carrier that is free of Ca²⁺ and Mg²⁺ cations; whereby the pharmaceutical composition is produced.

Another aspect of the disclosure is a method for increasing the closure of a skin wound on an animal comprising the steps of providing a pharmaceutical composition comprising a delta-PKC activator, an alpha-PKC inhibitor, and a pharmaceutically acceptable carrier that is free of Ca²⁺ and Mg²⁺ cations; and administering to a skin wound on an animal an effective amount of the pharmaceutical composition; whereby closure of the skin wound is increased.

Another aspect of the disclosure is a method for decreasing inflammation at the site of a skin wound on an animal comprising the steps of providing a pharmaceutical composition comprising a delta-PKC activator, an alpha-PKC inhibitor, and a pharmaceutically acceptable carrier that is free of Ca²⁺ and Mg²⁺ cations; and administering to a skin wound on an animal an effective amount of the pharmaceutical composition; whereby inflammation at the site of the skin wound is decreased.

Another aspect of the disclosure is a composition comprising an insulin or an insulin analog and a pharmaceutically acceptable carrier that is free of Ca²⁺ and Mg⁺² cations.

Another aspect of the disclosure is a composition comprising about 0.0001 units/L to about 0.1 units/L of an insulin and a pharmaceutically acceptable carrier that is free of Ca²⁺ and Mg²⁺ cations.

Another aspect of the disclosure is a method for increasing the closure of a wound on an animal comprising the steps of providing a pharmaceutical composition comprising a delta-PKC activator, an alpha-PKC inhibitor, and a pharmaceutically acceptable carrier that is free of Ca²⁺ and Mg²⁺ cations; and administering to a wound on an animal an effective amount of the pharmaceutical composition, wherein the wound is at least one selected from the group consisting of diabetic ulcer wounds, acral lick wounds, proud flesh wounds, surgical wounds, chronic solar abscess wounds, and osteomyelitis wounds; whereby closure of the wound is increased.

Another aspect of the disclosure is a composition comprising a delta-PKC activator, an alpha-PKC inhibitor, and a pharmaceutically acceptable carrier that contains K⁺ cations and is free of Ca²⁺ and Mg²⁺ cations.

Another aspect of the disclosure is a composition comprising a delta-PKC activator and a pharmaceutically acceptable carrier that contains K⁺ cations and is free of Ca²⁺ and Mg²⁺ cations.

Another aspect of the disclosure is composition comprising an alpha-PKC inhibitor, and a pharmaceutically acceptable carrier that is free of Ca²⁺ and Mg²⁺ cations.

Another aspect of the disclosure is a method for decreasing inflammation at the site of a skin wound on an animal comprising the steps of providing a pharmaceutical composition comprising an alpha-PKC inhibitor and a pharmaceutically acceptable carrier that is free of Ca²⁺ and Mg²⁺ cations; and administering to a skin wound on an animal an effective amount of the pharmaceutical composition; whereby inflammation at the site of the skin wound is decreased.

Other aspects of the invention include promoting the formation of granulation tissue, epidermal proliferation, and skin growth using compositions of the invention such as described herein.

Last, the compositions disclosed herein can be entirely free of Ca²⁺ and Mg²⁺ cations or contain pharmaceutically acceptable carriers that are free of these cations.

### BRIEF DESCRIPTION OF THE FIGURES

Figure 1A provides photos of cell culture dishes showing the efficacy of wound healing *in vitro* utilizing the indicated pharmaceutical compounds formulated in various formulations (Magnification of x50 under an Axiovert 25 Zeiss Microscope).
Figure 1B shows wound closure as a percent of closure 24 hours following treatment.
Figure 2A is a graph showing the pharmaceutical composition promotes significant wound closure in Formulation A.
Figure 2B are photos of representative wounds after treatment with various formulations.
Figure 3 is a graph showing the inflammatory burden at wound sites after treatment in various formulations.
Figure 4 is a graph showing granulation tissue formation after treatment with various formulations.
Figure 5 is a graph showing the ability of Myr-pseudosubstrate PKCα peptide to inhibit PKCα activity in various formulations.
Figure 6A are magnified photographs (Magnification of x200 under an Axiovert 25 Zeiss Microscope) of cell culture dishes showing the effects of insulin in various formulations on wound closure and cell proliferation.
Figure 6B is a graph showing wound closure *in vitro* as a percent of control 24 hours following treatment with the various formulations in the presence and absence of insulin.
Figure 6C is a graph showing cell proliferation as measured by thymidine incorporation.
Figure 7 is a graph showing the effects of Insulin and Insulin + PKCα inhibitor on cell proliferation in keratinocyte cells from 7 month old to 2 year old mice before and after changing the cell culture medium.
Figure 8A provides photos and graphs showing treatment of and increased closure of chronic foot ulcers with pharmaceutical composition in various formulations.
Figure 8B are photos showing treatment and increased closure of chronic diabetic wounds of a patient at day 0 and day 60 in various formulations.
Figure 9 provides photographs at day 0, 3 months and 6 months showing treatment of chronic Proud Flesh wounds in a horse with the pharmaceutical composition.
Figure 10 provides photographs at day 0, 30 and 60 showing treatment of chronic solar abscess with osteomyelitis with the pharmaceutical composition.
Figure 11 provides photographs at day 0, 2 months and 3.5 months showing the progress of treatment of non-healing acral lick wounds caused by self trauma with the pharmaceutical composition.

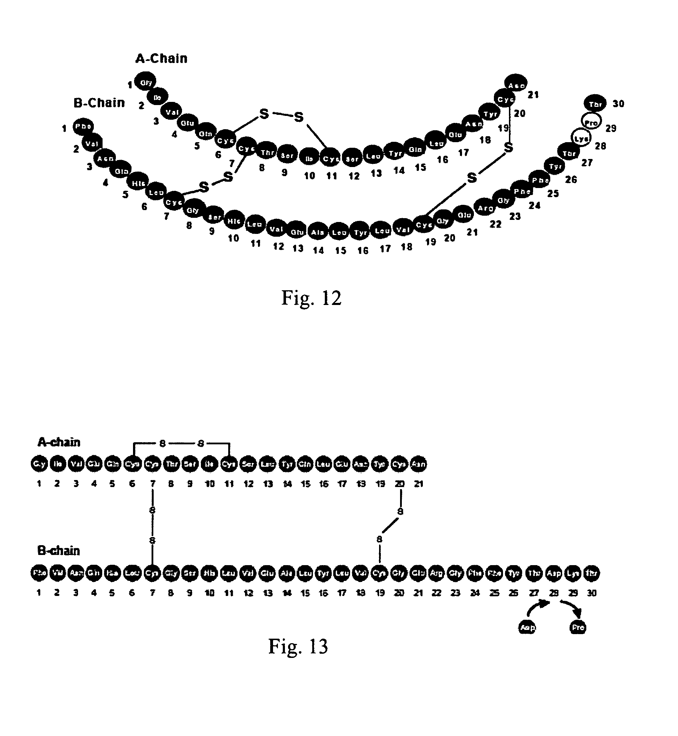

Figure 12 is a schematic representation of the primary structure of the human insulin analog, insulin lispro (rDNA origin) known by the trademark HUMALOG®.
Figure 13 is a schematic representation of the primary structure of the human insulin analog insulin aspart (rDNA origin), known by the trademark NOVOLOG®.

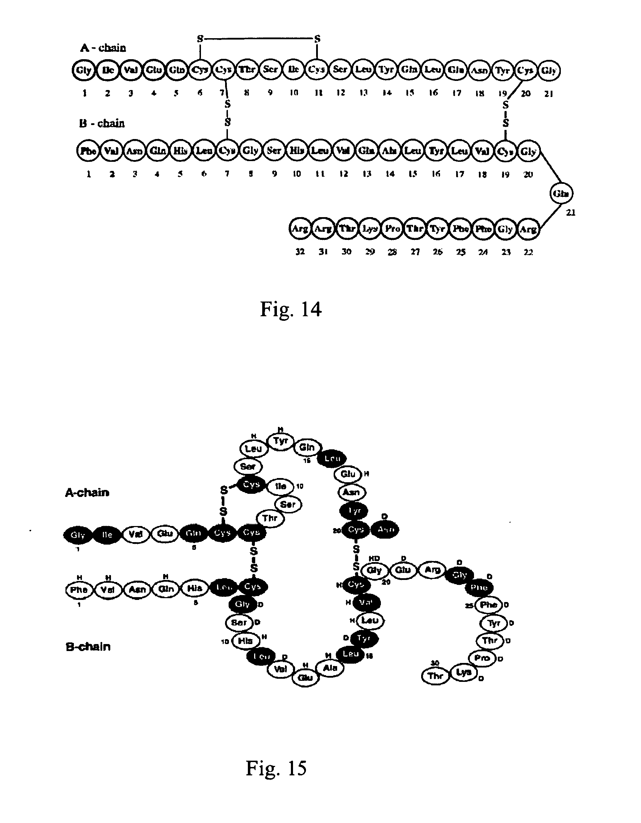

Figure 14 is a schematic representation of the primary structure of the human insulin analog insulin glargine (rDNA origin) known by the trademark LANTUS®.
Figure 15 is a schematic representation of the primary structure of the human insulin analog HUMULIN® R also known by the trademark NOVOLIN® R.
Figure 16 is a graph showing the percent of wound healing measured by formation of epidermis and granulation tissue after treatment with an insulin analog alone provided in Formulation A and compared to untreated control wounds. The insulin analogs studied were insulin lispro (HumL), insulin aspart (Novo), insulin glargine (LANTUS®), and HUMULIN® R (HumR).
Figure 17 is a graph showing the promotion of wound healing measured by the formation of granulation tissue with treatment of HUMULIN® R (HumR), USP Insulin (Ins USP), and PKCα pseudosubstrate inhibiting peptide (pep) alone or in a combination with an insulin analog and the inhibiting peptide.
Figure 18 is a graph showing the percent of severe inflammation with treatment of HUMULIN® R (HumR), insulin lispro (HumL), and PKCα pseudosubstrate inhibiting peptide (pep) alone or in a synergistic combination with an insulin analog and the inhibiting peptide.
Figure 19 is a graph showing keratin 1 in keratinocyte cells from 7 month old to 2 year old mice expression after treatment of visfatin or L-α-phosphatidylinositol-3,4,5-trisphosphate, dipalmitoyl-, heptaammonium salt in primary skin keratinocytes cultured in medium A and medium B.

### DETAILED DESCRIPTION OF THE DISCLOSURE

The pharmaceutical composition of the present disclosure comprises a pharmaceutically acceptable carrier comprising different inorganic and organic salts in variant solvents and a PKCα inhibitor, and/or insulin.

An exemplary formulation composition of a pharmaceutically acceptable carrier may contain water, potassium, sodium chloride, and phosphate at physiologically tolerable and can be prepared as follows:
A) Potassium Chloride 0.2 g/L (KCl)
B) Potassium Phosphate Monobasic (Anhydrous) 0.2 g/L (KH₂PO₄)
C) Sodium Chloride 8.0 (g/L) (NaCl)
D) Sodium Phosphate Dibasic (anhydrous) 1.15 (g/L) (Na₂HPO₄)
The formulation must not contain calcium or magnesium ions.

While any PKCα inhibitor can be used, preferably, the PKCα inhibitor is a myristoylated peptide corresponding to the pseudosubstrate region of PKCα (Myr*-Phe-Ala-Arg-Lys-Gly-Ala-Leu-Arg-Gln-OH (SEQ ID NO: 1 CAS [147217-25-2]). The PKCα pseudosubstrate region has an especially high affinity to the substrate region of this particular isoform. Examples of additional PKC inhibitors that can be used include the peptides shown in Table 1 below.

**Table 1. PKC Inhibitor Peptides**

| | |
|---|---|
| Arg Phe Ala Arg Lys Gly Ala Leu Arg Gln Lys Asn Val | SEQ ID NO: 2 |
| Arg Phe Ala Arg Lys Gly Ala Leu Arg Gln Lys Asn Val His Glu Val Lys Asn | SEQ ID NO: 3 |
| Arg Phe Ala Arg Lys Gly Ala Leu Arg Gln Lys Asn Val His Glu Val Lys Asn Leu Lys Gly Ala | SEQ ID NO: 4 |
| Arg Phe Ala Arg Lys Gly Ala Leu Arg Gln Leu Ala Val | SEQ ID NO: 5 |
| Arg Phe Ala Arg Lys Gly Ala Leu Ala Gln Lys Asn Val | SEQ ID NO: 6 |
| Arg Phe Ala Arg Lys Gly Ala Leu Arg | SEQ ID NO: 7 |
| Tyr Tyr Xaa Lys Arg Lys Met Ala Phe Phe Glu Phe Phe (Xaa can be any naturally occurring amino acid) | SEQ ID NO: 8 |
| Phe Lys Leu Lys Arg Lys Gly Ala Phe Lys Lys Phe Ala | SEQ ID NO: 9 |
| Ala Arg Arg Lys Arg Lys Gly Ala Phe Phe Tyr Gly Gly | SEQ ID NO: 10 |
| Arg Arg Arg Arg Arg Lys Gly Ala Phe Arg Arg Lys Ala | SEQ ID NO: 11 |
| Arg Phe Ala Arg Lys Gly Ala Leu Arg Gln Lys Asn Val Tyr | SEQ ID NO: 12 |
| Asp Ala Arg Lys Gly Ala Leu Arg Gln Asn Lys Val | SEQ ID NO: 13 |
| Glu Arg Met Arg Pro Arg Lys Arg Gln Gly Ala Val Arg Arg Arg Val | SEQ ID NO: 14 |
| Gly Pro Arg Pro Leu Phe Cys Arg Lys Gly Ala Leu Arg Gln Lys Val Val | SEQ ID NO: 15 |
| Gln Lys Arg Pro Ala Gln Arg Ser Lys Tyr Leu | SEQ ID NO: 16 |
| Gln Lys Arg Pro Ser Gln Arg Ala Lys Tyr Leu | SEQ ID NO: 17 |
| Gly Gly Pro Leu Arg Arg Thr Leu Ala Val Arg Arg | SEQ ID NO: 18 |
| Gly Gly Pro Leu Ser Arg Arg Leu Ala Val Arg Arg | SEQ ID NO: 19 |
| Gly Gly Pro Leu Ser Arg Thr Leu Ala Val Arg Arg | SEQ ID NO: 20 |
| Gly Gly Pro Leu Ser Arg Arg Leu Ala Val Ala Arg | SEQ ID NO: 21 |
| Gly Gly Pro Leu Arg Arg Thr Leu Ala Val Ala Arg | SEQ ID NO: 22 |
| Val Arg Lys Ala Leu Arg Arg Leu | SEQ ID NO: 23 |
| Gly Gly Arg Leu Ser Arg Thr Leu Ala Val Ala Arg | SEQ ID NO: 24 |
| Thr Arg Lys Arg Gln Pro Ala Met Arg Arg Arg Val His Gln Ile Asn Gly | SEQ ID NO: 25 |
| This peptide is myristolated at the N terminus and amidated at the C-terminus. | |
| Arg Lys Arg Gln Arg Ala Met Arg Arg Arg Val His | SEQ ID NO: 26 |
| Glu Arg Met Arg Pro Arg Lys Arg Gln Gly Ala Val Arg Arg Arg Val | SEQ ID NO: 27 |
| Phe Lys Leu Lys Arg Lys Gly Ala Phe Lys Lys Phe Ala | SEQ ID NO: 28 |
| Tyr Tyr Xaa Lys Arg Lys Met Ala Phe Phe Glu Phe Phe | SEQ ID NO: 29 |
| Xaa can be any naturally occurring amino acid | |
| Ala Arg Arg Lys Arg Lys Gly Ala Phe Phe Tyr Gly Gly | SEQ ID NO: 30 |
| Arg Arg Arg Arg Arg Lys Gly Ala Phe Arg Arg Lys Ala | SEQ ID NO: 31 |
| Ala Ala Ala Lys Ile Gln Ala Ala Trp Arg Gly His Met Ala Arg Lys Lys Ile Lys Ser | SEQ ID NO: 32 |
| Ala Ala Ala Lys Ile Gln Ala Ala Phe Arg Gly His Met Ala Arg Lys Lys Ile Lys | SEQ ID NO: 33 |
| Glu Arg Met Arg Pro Arg Lys Arg Gln Gly Ala Val Arg Arg Arg Val | SEQ ID NO: 34 |
| Val Arg Lys Ala Leu Arg Arg Leu | SEQ ID NO: 35 |
| Lys Lys Lys Lys Lys Arg Phe Ser Phe Lys Lys Ala Phe Lys Leu Ser Gly Phe Ser Phe Lys Lys | SEQ ID NO: 36 |
| Gly Pro Arg Pro Leu Phe Cys Arg Lys Gly Ala Leu Arg Gln Lys Val Val | SEQ ID NO: 37 |
| Glu Ser Thr Val Arg Phe Ala Arg Lys Gly Ala Leu Arg Gln Lys Asn Val l | SEQ ID NO: 38 |
| Glu Arg Met Arg Pro Arg Lys Arg Gln Gly Ala Val Arg Arg Arg Val | SEQ ID NO: 39 |
| Arg Phe Ala Arg Leu Ala Leu Arg Gln Lys Asn Val | SEQ ID NO: 40 |
| Tyr Tyr Xaa Lys Arg Lys Met Ala Phe Phe Glu Phe Phe | SEQ ID NO: 41 |
| Xaa can be any naturally occurring amino acid | |
| Arg Arg Phe Lys Arg Gln Gly Ala Phe Phe Tyr Phe Phe | SEQ ID NO: 42 |
| Phe Lys Leu Lys Arg Lys Gly Ala Phe Lys Lys Phe Ala | SEQ ID NO: 43 |
| Ala Arg Arg Lys Arg Lys Gly Ser Phe Phe Tyr Gly Gly | SEQ ID NO: 44 |
| Phe Lys Leu Lys Arg Lys Gly Ser Phe Lys Lys Phe Ala | SEQ ID NO: 45 |
| Arg Arg Phe Lys Arg Gln Gly Ser Phe Phe Tyr Phe Phe | SEQ ID NO: 46 |
| Tyr Tyr Xaa Lys Arg Lys Met Ser Phe Phe Glu Phe Phe | SEQ ID NO: 47 |
| Xaa can be any naturally occurring amino acid | |
| Arg Arg Arg Arg Arg Lys Gly Ser Phe Arg Arg Lys Ala | SEQ ID NO: 48 |
| Glu Arg Met Arg Pro Arg Lys Arg Gln Gly Ser Val Arg Arg Arg Val | SEQ ID NO: 49 |
| Met Asn Arg Arg Gly Ser Ile Lys Gln Ala Lys Ile | SEQ ID NO: 50 |
| Met Phe Ala Val Arg Asp Arg Arg Gln Thr Val Lys Lys Gly Val Ile Lys Ala Val Asp Ala Val | SEQ ID NO: 51 |
| Phe Gly Glu Ser Arg Ala Ser Thr Phe Cys Gly Thr Pro Asp | SEQ ID NO: 52 |
| Lys Ala Arg Leu Ser Tyr Ser Asp Lys Asn | SEQ ID NO: 53 |
| Ser Ala Phe Ala Gly Phe Ser Phe Val Asn Pro Lys Phe | SEQ ID NO: 54 |
| Lys Lys Lys Lys Lys Arg Phe Ser Phe Lys Lys Ser Phe Lys Leu Ser Gly Phe Ser Phe Lys Lys | SEQ ID NO: 55 |

In addition, the following PKC inhibitors can also be used in a pharmaceutical composition according to the present disclosure:
A) NPC 15437 - dihydrochloride hydrate (Sigma), also known as (*S*)-2,6-diamino-N-[(1-(1-oxotridecyl)-2-piperidinyl)methyl]hexanamide dihydrochloride hydrate.
   Molecular Formula - C₂₅H₅₀N₄O₂ · 2HCl · xH₂O
   Molecular Weight - 511.61 (anhydrous basis)
   CAS Number - 141774-20-1 (anhydrous)
   MDL number - MFCD00210207
   PubChem Substance ID - 24897504
B) CGP41251 - [4'-N-Benzoyl Staurosporine] [Midostaurin]. The staurosporine derivative PKC 412(CGP 41251) is a more selective inhibitor of the conventional isoforms of protein kinase C(PKC).
   Molecular Formula - C₃₅H₃₀N₄O₄
   Molecular Weight - 570.65 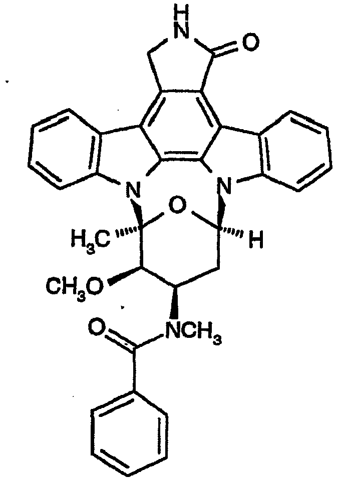
C) Ro 31-8220 - Bisindolylmaleimide IX, Methanesulfonate salt. (Upstate Bitotechnology)
   Molecular Formula - C₂₅H₂₃N₅O₂S·CH₄O₃S
   Molecular Weight - 553.66
   Catalog # 19-163; the formula is shown below: 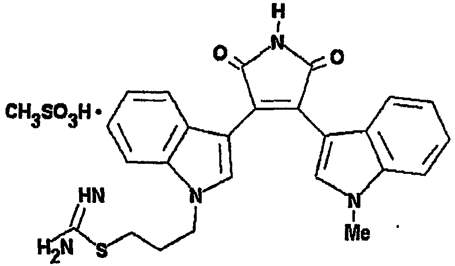
D) Gö6976 which is 12-(2-cyanoethyl)-6,7,12,13-tetrahydro-13-methyl- 5-oxo-5H-indolo[2,3-a]pyrrollo[3,4-c]carbazole, an alpha and PKC betal inhibitor.
E) GF-109203X 2-[1-(3-Dimethylaminopropyl)-1H-indol-3-yl]-3-(1H-indol-3-yl) maleimide, a potent and selective protein kinase C inhibitor.
F) ISIS 3521/LY900003, also known as aprinocarsen, 20-nucleotide phosphorothioate de-oxyribo-oligonucleotide commercially available from Isis Pharmaceuticals, Inc., Carlsbad, CA, with the following sequence (SEQ ID NO: 56):
   5'-GTTCTCGCTGGTGAGTTTCA-3'

In a preferred embodiment, the pharmaceutical composition of the present disclosure comprises a pharmaceutically acceptable carrier, regular insulin or a functional analog thereof which activates PKCδ, and a commercially available synthetic peptide composed of 9 amino acids, which inhibits PKCα.

A preferred pharmaceutical composition, comprises:
a) Potassium Chloride 0.2 g/L (KCl)
b) Potassium Phosphate Monobasic (Anhydrous) 0.2 g/L (KH₂PO₄)
c) Sodium Chloride 8.0 (g/L) (NaCl)
d) Sodium Phosphate Dibasic (anhydrous) 1.15 (g/L) (Na₂HPO₄)
e) Myristoylated peptide (1-100 µM) such as

Myr*-Phe-Ala-Arg-Lys-Gly-Ala-Leu-Arg-Gln-OH (SEQ ID NO: 1) f) Regular Insulin or a functional analog thereof (therapeutic dose: 0.1-10 units/ml) The concentrations listed above are preferred the final concentrations in the composition.

The pharmaceutical composition is prepared by mixing insulin or a functional analog thereof with a PKCα inhibitor in a pharmaceutically acceptable carrier that does not contain calcium or magnesium ions. It is contemplated that a pharmaceutical composition according to this disclosure can be prepared in the form of a solution, a gel, an ointment, a cream, or an emulsion by methods readily available to one of skill in the art.

The two bioactive components, insulin and PKCα inhibitor peptide act together to induce wound healing when formulated in a solution. The concentration of insulin or a functional insulin analog may be 0.1-10 units/mL. The concentration of the peptide inhibitor of PKCα may be 1 to 100 µM. A preferred concentration is 0.1 unit of insulin (10⁻⁶ M) and 1 µg of peptide (10⁻⁶ M) in 1 ml of solution.

The insulin for use in a pharmaceutical composition according to present disclosure may be recombinant or from a natural source such as human insulin or a non-human mammal insulin that is suitable for human use. It is also contemplated that the pharmaceutical composition may be prepared with an insulin analog such as a functional analog of insulin. Non-limiting examples of insulin analogs are insulin lispro, insulin aspart, insulin glargine, and recombinant human insulin, visfatin, and L-α-phosphatidylinositol-3,4,5-trisphosphate, dipalmitoyl-, heptaammonium salt (also identified herein as L-alpha).

Certain of these insulin analogs share a basic primary structure similar to the structure of regular human insulin. Insulin lispro is distinguished from human insulin because the proline at B-28 and the lysine at B-29 are reversed in the analog. Insulin aspart is distinguished from human insulin because the proline at B-28 is substituted with aspartic acid. Insulin glargine is distinguished from human insulin because the amino acid asparagine at position A-21 is replaced by glycine, and two arginine residues are added to the C-terminus of the β-chain. Recombinant human insulin can be structurally identical to human insulin and is produced by rDNA technology, such as by using *Saccharomyces cerevisiae* to produce the peptides.

Visfatin is an adipocytokine that functions as an insulin analog and is an insulin mimetic capable of binding to and activating the insulin receptor. L-alpha is an organic compound that activates Ca²⁺-insensitive PKC isozymes δ, ε, and η. It binds to the general receptor for phosphoinositide-1 (GRP1) protein through a plekstrin homology (PH) domain and is also reported to increase the motility of NIH/3T3 cells and produce actin reorganization and membrane ruffling.

In a preferred embodiment, a therapeutically effective amount of the pharmaceutical composition is administered to a subject in need thereof. The pharmaceutical composition can be administered by any known route of administration effective to provide the desired therapy, preferably by topical application in a solution, ointment, gel, cream or any local application (such as subcutaneous injection). The pharmaceutical composition may also be administered by means of a drug eluting device, such as gauze, a patch, pad, or a sponge.

A further aspect of the present pharmaceutical composition according to this disclosure is treating damaged skin or a skin wound using the pharmaceutical composition. The composition should be administered as frequently as necessary and for as long of a time as necessary to treat the wound in order and achieve the desired endpoint, *e.g.*, until the wound completely resolves. One of ordinary skill in the art can readily determine a suitable course of treatment utilizing the compositions and methods according to this disclosure.

Further aspects of a pharmaceutical composition according to this disclosure are promoting the formation of granulation tissue, epidermal proliferation, and skin growth. Another aspect of the pharmaceutical composition according to this disclosure is a method of treating inflammation, such as inflammation caused by inflammatory skin disease.

The term "alpha-PKC inhibitor" as used herein means a molecule that can inhibit the activity of a PKCα isoform by any mechanism. Examples of PKCα isoforms include the PKCα isoforms encoded by the nucleic acids described in Accession Numbers NM_002737 (*Homo sapiens* PKCα), XM_548026 (*Canis lupus familiaris* PKCα), XM_001494589 (*Equus caballus* PKCα), and NM_011101 (*Mus musculus* PKCα) or peptide chains that are at least 95% identical to the mature form of these PKCα isoforms as determined using the default settings of the CLUSTALW algorithm. Alpha-PKC inhibitor molecules can inhibit PKCα isoforms directly by binding, covalent modification or other mechanisms involving physical interaction of such molecules with a PKCα isoform. Alpha-PKC inhibitor molecules can also inhibit PKCα isoforms indirectly by modulating the activity of a second molecule involved in the activation of a PKCα isoform (*e.g.* by modulating the activity of a component of a PKCα isoform related signaling cascade to inhibit the activity of PKCα isoforms or by silencing RNAs that prevent expression of PKCα isoforms).

The term "delta-PKC activator" as used herein means as used herein means a molecule that can activate a PKCδ isoform, or increase the PKCδ isoform activity in a cell or tissue, by any mechanism. Examples of PKCδ isoforms include the PKCδ isoforms encoded by the nucleic acids described in Accession Numbers NM_006254 *(Homo sapiens* PKCδ), NM_001008716 (*Canis lupus familiaris* PKCδ), XM_001915127 (*Equus caballus* PKCδ), and NM_011103 (*Mus musculus* PKCδ) or peptide chains that are at least 85% identical to the mature form of these PKCδ isoforms as determined using the default settings of the CLUSTALW algorithm. Delta-PKC activator molecules can activate PKCδ isoforms directly by binding, covalent modification or other mechanisms involving physical interaction of such molecules with a PKCδ isoform and can include PKCδ isoform substrates and cofactors. Delta-PKC activator molecules can also activate PKCδ isoforms indirectly by modulating the activity of a second molecule involved in the activation of a PKCδ isoform (*e.g*. by modulating the activity of a component of a PKCδ isoform related signaling cascade, such as an insulin receptor to activate a PKCδ isoform). Delta-PKC activator molecules can also increase the PKCδ isoform activity in a cell or tissue by producing increased expression of PKCδ isoforms in a cell or tissue.

The term "drug eluting scaffold" as used herein means a stationary material capable of releasing a physiologically active molecule. Drug eluting scaffolds may comprise stationary phase materials which may be insoluble, soluble, non-bioabsorbable, or bioabsorbable.

The term "insulin" as used herein means those naturally occurring peptide hormones and their preproinsulin and proinsulin precursor forms that comprises in their mature form disulfide bond linked A and B chains which can activate an insulin receptor and are known to be useful in the treatment of diabetes. Insulins from a number of different animal species such as humans, cows, and pigs are well known and will be readily recognized by those of ordinary skill in the art. Importantly, insulins can be recombinantly produced.

The term "insulin analog" as used herein means a molecule comprising a structure not found in naturally occurring insulins which can activate an insulin receptor by any mechanism. Such molecules can be structural analogs of insulins in which one or more structural aspects of a naturally occurring insulin have been modified. Such molecules can also be mimetic molecules which do not comprise structures found in a naturally occurring insulin. Insulin analogs can also include insulin-like growth factors (*e.g*. insulin-like growth factor-1). Insulin analogs can activate an insulin receptor directly by binding, covalent modification or other mechanisms involving physical interaction with such receptors. Insulin analogs can also activate insulin receptors indirectly by modulating the activity of a second molecule involved in the activation of such receptors. Without wishing to be bound be theory it is believed that activation of insulin receptors results in the indirect activation of PKCδ isoforms. A number of different insulin analogs are well known and will be readily recognized by those of ordinary skill in the art.

The term "standard state" as used herein means a temperature of 25°C +/- 2°C and a pressure of 1 atmosphere. The concentrations of the solutions, suspensions, and other preparations described herein and expressed on a per unit volume basis (*e.g*. mol/L, M, units/ml, µg/ml *etc*.) are determined at "standard state." The term "standard state" is not used in the art to refer to a single art recognized set of temperatures or pressure, but is instead a reference state that specifies temperatures and pressure to be used to describe a solution, suspension, or other preparation with a particular composition under the reference "standard state" conditions. This is because the volume of a solution is, in part, a function of temperature and pressure. Those skilled in the art will recognize that compositions equivalent to those disclosed here can be produced at other temperatures and pressures.

The term "pharmaceutically acceptable carrier" as used herein means one or more compatible solid or liquid filler diluents or encapsulating substances which are suitable for administration to a human or other animal.

One aspect of the disclosure is a composition comprising a delta-PKC activator, an alpha-PKC inhibitor, and a pharmaceutically acceptable carrier that is free of Ca²⁺ and Mg²⁺ cations. Ideally, pharmaceutically acceptable carriers should be of high purity and low toxicity to render them suitable for administration to the human or animal being treated. Such pharmaceutically acceptable carriers should also maintain the biological activity of a delta-PKC activator and an alpha-PKC inhibitor.

Such pharmaceutically acceptable carriers can also include, for example, acetate based buffers, 2-morpholinoethansulfonic (MES) based buffers, potassium hydrogen phthalate based buffers, KH₂PO₄ based buffers, tris(hydroxymethyl) aminomethane based buffers, and borax (Na₂B4O₇ 10H₂O) based buffers. 100 mL 0.1 M potassium hydrogen phthalate + volume indicated (in mL) 0.1 M NaOH. Such buffers can be made, or can comprise, the following recipes:
100 mL of 0.1 M KH₂O₄ adjusted to the desired pH with 0.1 M NaOH;
100 mL 0.1 M tris (hydroxymethyl) aminomethane adjusted to the desired pH with 0.1 M HCl; and
100 mL 0.025 M Na₂B₄O₇ 10H₂O (borax) adjusted to the desired pH with 0.1 M HCl.

Examples of suitable pharmaceutically acceptable carriers include water, petroleum jelly, petrolatum, mineral oil, vegetable oil, animal oil, organic and inorganic waxes, such as microcrystalline, paraffin and ozocerite wax, natural polymers such as xanthanes, malt, talc, gelatin, sugars, cellulose, collagen, starch, or gum arabic, synthetic polymers, alcohols, polyols, phosphate buffer solutions, cocoa butter, emulsifiers, detergents such as the TWEENs™ and the like. The carrier may be a water miscible carrier composition that is substantially miscible in water such as, for example, alcohols. Water miscible topical pharmaceutically acceptable carriers can include those made with one or more ingredients described above, and can also include sustained or delayed release carriers, including water containing, water dispersible or water soluble compositions, such as liposomes, microsponges, microspheres or microcapsules, aqueous base ointments, water-in-oil or oil-in-water emulsions, gels or the like. Those of ordinary skill in the art will recognize other pharmaceutically acceptable carriers.

Other compatible pharmaceutical actives and additives may be included in the pharmaceutically-acceptable carrier for use in the compositions of the present invention. For example, local anesthetics such as NOVOCAINE™, lidocaine, or others may be included in the pharmaceutically acceptable carrier. Additives such as benzyl alcohol and other preservatives may also be included in the pharmaceutically acceptable carrier. Those of ordinary skill in the art will readily recognize other pharmaceutically acceptable actives and additives.

In some embodiments of the compositions and methods of the disclosure the delta-PKC activator is at least one selected from the group consisting of an insulin and an insulin analog.

In some embodiments of the compositions and methods of the disclosure the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt. Examples of other insulin analogs include insulin glulisine, insulin detemir, and albulin. Certain of these insulin analogs are also known by the tradenames APIDRA®, HUMALOG®, LANTUS®, LEVEMIR®, NOVOLIN®, HUMULIN®, NOVOLOG®. Moreover, HUMULIN® R can be formulated to comprise 0.16 mg/ml glycerin and 0.7 µg/ml zinc chloride. The pH of these HUMULIN® R compositions can be adjusted to pH 7.4 with 1 N hydrochloric acid or 1 N sodium hydroxide. The compositions disclosed herein can also comprise the components of the HUMULIN® R insulin analog formulation, including the Zn²⁺ ion, described above.

Visfatin can comprise the *Homo sapiens* visfatin amino acid sequences shown in SEQ ID NO: 63. Visfatin can also comprise the *Mus musculus* visfatin amino acid sequence shown in SEQ ID NO: 64. Those skilled in the art will recognize other visfatin molecules such as those molecules having greater than 90% identity, or greater than 95% identity to SEQ ID NO: 63 or SEQ ID NO: 64 or biologically active fragments or variants of these. Additionally, those of ordinary skill in the art will recognize that amino terminal methionine residues are typically excised from the mature form of polypeptide chains such as visfatin and others expressed *in vivo.*

In some embodiments of the compositions and methods of the disclosure the insulin is at least one selected from the group consisting of human insulin, bovine insulin, and porcine insulin.

In some embodiments of the compositions and methods of the disclosure the insulin is recombinantly expressed. Recombinant expression by transformation of a host cell with recombinant DNA may be carried out by conventional techniques which are well known to those skilled in the art. The host cell may be a prokaryotic, archaeal, or eukaryotic cell. The isolation and purification of recombinantly expressed polypeptides such as recombinant insulin peptide chains can carried out by techniques that are well known in the are including, for example, preparative chromatography and affinity purification using antibodies or other molecules that specifically bind a given polypeptide.

In some embodiments of the compositions and methods of the disclosure the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen. Those of ordinary skill in the art will recognize that in the disclosed compositions the PKC inhibitors can be in the form of salts, hydrates, and complexes. Additionally, one of ordinary skill in the art will recognize that PKC inhibitors can be combined in the disclosed compositions.

In some embodiments of the compositions and methods of the disclosure the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.

Such peptides can be synthesized by such commonly used methods as t-BOC or FMOC protection of alpha-amino groups. Both methods involve stepwise syntheses whereby a single amino acid is added at each step starting from the carboxy terminus of the peptide (Coligan et al., Current Protocols in Immunology, Wiley Interscience, 1991, Unit 9). Peptides of the invention can also be synthesized by the well known solid phase peptide synthesis methods described in Merrifield (85 J. Am. Chem. Soc. 2149 (1962)), and Stewart and Young, Solid Phase Peptides Synthesis, (Freeman, San Francisco, 1969, pp.27-62), using a copoly(styrenedivinylbenzene) containing 0.1-1.0 mMol amines/g polymer. On completion of chemical synthesis, the peptides can be deprotected and cleaved from the polymer by treatment with liquid HF-10% anisole for about 1/4-1 hours at 0°C. After evaporation of the reagents, the peptides are extracted from the polymer with a 1% acetic acid solution which is then lyophilized to yield the crude material. This can normally be purified by such techniques as gel filtration on Sephadex G-15 using 5% acetic acid as a solvent. Lyophilization of appropriate fractions of the column will yield the homogeneous peptide or peptide derivatives, which can then be characterized by such standard techniques as amino acid analysis, thin layer chromatography, high performance liquid chromatography, ultraviolet absorption spectroscopy, molar rotation, and solubility based methods.

Peptides can also be synthesized by any biological method, such as by recombinant expression of the protein in mammalian cells, insect cells, yeast and bacteria and cell free systems such as *in vitro* transcription and translation systems. Protein expression can be optimized for each system by well-established methods. Protein can be purified by standard methods (Frederich M. Ausubel, et al., Current Protocols in Molecular Biology, Wiley Interscience, 1989). For example, the protein can be expressed in bacteria as GST-fusion protein and purified by glutathione agarose beads (Sigma) as described (Erangionic and Neel, Analytical Biochemistry, 210:179, 1993). Alternatively, the protein can be expressed as a secretory product in mammalian cells and purified from conditioned medium (Cadena and Gill, Protein Expression and Purification 4:177, 1993). Peptides prepared by the method of Merrifield can be synthesized using an automated peptide synthesizer such as the Applied Biosystems 431A-01 Peptide Synthesizer (Mountain View, Calif.) or using the manual peptide synthesis technique described by Houghten, Proc. Natl. Acad. Sci., USA 82:5131 (1985). Peptides may also be synthesized by, using covalent modification, liquid-phase peptide synthesis, or any other method known to one of ordinary skill in the art.

Peptides can be synthesized using amino acids or amino acid analogs, the active groups of which are protected as necessary using, for example, a t-butyldicarbonate (t-BOC) group or a fluorenylmethoxy carbonyl (FMOC) group. Amino acids and amino acid analogs can be purchased commercially (Sigma Chemical Co.; Advanced Chemtec) or synthesized using methods known in the art.

Amino acids in the peptides disclosed herein can be modified by amino acid substitution of one or more of the specific amino acids shown in the exemplified peptides. An amino acid substitution change can include the substitution of one basic amino acid for another basic amino acid, one hydrophobic amino acid for another hydrophobic amino acid or other conservative substitutions. Amino acid substitutions can also include the use of non-naturally occurring amino acids such as, for example, ornithine (Orn) or homoArginine (homoArg) for Arg.

Peptides can also be modified by the covalent attachment of other molecules or reaction of a functional group present in a peptide. Examples of such modifications include the attachment of polyethyleneglycol molecules, lipid, carbohydrate, or other molecules. Specific examples of such modifications also include myristoylation and amidation. Techniques for the covalent modification of peptides are well known in the art and those of ordinary skill will recognize a number of such techniques.

In some embodiments of the compositions and methods of the disclosure the alpha-PKC inhibitor is a peptide consisting of the amino acid sequence shown in SEQ ID NO: 25 which has a myristoylated amino acid residue at its amino terminus and is amidated at its carboxy terminus.

In some embodiments of the compositions and methods of the disclosure the alpha-PKC inhibitor is a peptide consisting of the amino acid sequence shown in SEQ ID NO: 1 which has a myristoylated amino acid residue at its amino terminus.

In some embodiments of the compositions and methods of the disclosure the pharmaceutically acceptable carrier that is free of Ca²⁺ and Mg²⁺ cations is an aqueous carrier comprising 0.2 g/L KCI, 0.2 g/L anhydrous KH₂PO₄, 8 g/L NaCl, and 1.15 g/L anhydrous Na₂HPO₄.

Another aspect of the disclosure is a composition comprising an insulin, a peptide consisting of the amino acid sequence shown in SEQ ID NO: 1 which has a myristoylated amino acid residue at its amino terminus, and an aqueous pharmaceutically acceptable carrier comprising 0.2 g/L KCI, 0.2 g/L anhydrous KH₂PO₄, 8 g/L NaCl, and 1.15 g/L anhydrous Na₂HPO₄ that is free of Ca²⁺ and Mg²⁺ cations.

In some embodiments of the compositions and methods of the disclosure the composition comprises about 0.0001 units/L to about 0.1 units/L of insulin and about 1 µM to about 100 µM of the peptide.

In some embodiments of the compositions and methods of the disclosure the composition comprises 0.0001 units/L of insulin and 1 µM of the peptide.

Another aspect of the disclosure is a composition comprising a delta-PKC activator, an alpha-PKC inhibitor, a pharmaceutically acceptable carrier that is free of Ca²⁺ and Mg²⁺ cations, and a drug eluting scaffold. The drug eluting scaffold may be any solid phase structure capable of delivering a pharmaceutical composition. The drug eluting scaffold may retain the pharmaceutical composition and deliver it over time by means such as diffusion, capillary action, gravity, or other physical processes for mobilizing molecules. The drug eluting scaffold may comprise, for example, layered or woven fibers, a fibrous mat, a foam, gels, a matrix of different solids or any other solid phase structure and can be provided in any form such as a stent. Those of ordinary skill in the art will recognize other suitable drug eluting scaffolds.

In one embodiment of the composition the drug eluting scaffold comprises a porous solid. Examples of such porous solids include sponges, foams, gauzes, gels, or other matrices. Those skilled in the art will recognize other examples of drug eluting scaffolds.

In one embodiment of the compositions the drug eluting scaffold is a sponge.

Another aspect of the disclosure is a pharmaceutical composition produced by a process comprising the steps of a) providing a delta-PKC activator, an alpha-PKC inhibitor, and a pharmaceutically acceptable carrier that is free of Ca²⁺ and Mg²⁺ cations; and b) combining the delta-PKC activator, alpha-PKC inhibitor, and the pharmaceutically acceptable carrier that is free of Ca²⁺ and M²⁺ cations; whereby the pharmaceutical composition is produced.

The other compositions disclosed herein can also be produced by processes that similarly involve the steps of providing the components of the compositions and then combining these components to produced such compositions.

Another aspect of the disclosure is a method for increasing the closure of a skin wound on an animal comprising the steps of a) providing a pharmaceutical composition comprising a delta-PKC activator, an alpha-PKC inhibitor, and a pharmaceutically acceptable carrier that is free of Ca²⁺ and Mg²⁺ cations; and b) administering to a skin wound on an animal an effective amount of the pharmaceutical composition; whereby closure of the skin wound is increased.

Closure of a skin wound can be assessed by identifying the unaffected margins of a wound that comprises normal tissue and determining the area within the margins of the wound that is unhealed. The closure of a wound occurs when the unhealed area within the margins of a wound decreases relative a prior measurement. Ultimately, increasing closure of a skin wound results in the total closure of a wound such that there is no unhealed area. Those of ordinary skill in the art will recognize other techniques for assessing wound closure and whether it is increasing.

One of ordinary skill in the art can determine an effective amount of the pharmaceutical composition by histology, H & E staining, keratin 14 staining, or immunochemistry or by observing abscess formation, excessive leukocytosis, and high RBC/WBC ratio in blood vessels by routine experimentation easily performed by one of ordinary skill in the art. One of skill in the art can also identify that an effective amount of the pharmaceutical composition has been administered to a subject with a skin wound by simply observing or measuring the change in area of the wound before treatment and a reasonable time after treatment.

Pharmaceutical compositions suitable for administration in the methods of the disclosure may be provided in the form of solutions, ointments, emulsions, creams, gels, granules, films and plasters. Those of ordinary skill in the art will recognize other forms of the disclosed pharmaceutical compositions suitable for administration.

Another aspect of the invention is a method for decreasing inflammation at the site of a skin wound on an animal comprising the steps of a) providing a pharmaceutical composition comprising a delta-PKC activator, an alpha-PKC inhibitor, and a pharmaceutically acceptable carrier that is free of Ca²⁺ and Mg²⁺ cations; and b) administering to a skin wound on an animal an effective amount of the pharmaceutical composition; whereby inflammation at the site of the skin wound is decreased.

Inflammation occurs when at least two of the following parameters were present at the site of skin wound abscess formation at the wounded area, excessive leukocytosis (>100 cells in a fixed field x200), and high WBC/RBC (white blood cell/red blood cell) ratio in blood vessels where >20% of WBC content within the blood vessels is shown in a fixed field (x200). Inflammation can be considered to be decreased when none or only one of the above parameters is present at the site of a skin wound. Alternatively, inflammation at a skin wound site can be assessed by other well known clinical signs such as swelling, redness, puss and the like. Inflammation can be considered to be decreased when the severity of these clinical signs is decreased or entirely ablated. Those of ordinary skill in the art will also recognize other techniques for assessing inflammation and whether it is decreasing.

Another aspect of the disclosure is a composition comprising an insulin or an insulin analog and a pharmaceutically acceptable carrier that is free of Ca²⁺ and Mg²⁺ cations.

In some embodiments of the compositions and methods of the disclosure the composition comprises about 0.0001 units/L to about 0.1 units/L of an insulin or an insulin analog.

In some embodiments of the compositions and methods of the disclosure the composition comprises about 0.0001 units/L of an insulin or an insulin analog.

In some embodiments of the compositions and methods of the disclosure the composition comprises about 0.0001 units/L to about 0.1 units/L of an insulin and a pharmaceutically acceptable carrier that is free of Ca²⁺ and Mg²⁺ cations.

Another aspect of the disclosure is a method for increasing the closure of a wound on an animal comprising the steps of providing a pharmaceutical composition comprising a delta-PKC activator, an alpha-PKC inhibitor, and a pharmaceutically acceptable carrier that is free of Ca²⁺ and Mg²⁺ cations; and administering to a wound on an animal an effective amount of the pharmaceutical composition, wherein the wound is at least one selected from the group consisting of diabetic ulcer wounds, acral lick wounds, proud flesh wounds, surgical wounds, chronic solar abscess wounds, and osteomyelitis wounds; whereby closure of the wound is increased.

Another aspect of the disclosure is a composition comprising a delta-PKC activator, an alpha-PKC inhibitor, and a pharmaceutically acceptable carrier that contains K⁺ cations and is free of Ca²⁺ and Mg²⁺ cations. Examples of sources of K⁺ cations include potassium chloride (KCl), potassium bicarbonate (KHCO₃), and potassium phosphate (KH₂PO₄). Those of ordinary skill in the art will readily recognize other sources of K⁺ cations.

Another aspect of the disclosure is a composition comprising a delta-PKC activator and a pharmaceutically acceptable carrier that contains K⁺ cations and is free of Ca²⁺ and Mg²⁺ cations.

Another aspect of the disclosure is composition comprising an alpha-PKC inhibitor, and a pharmaceutically acceptable carrier that is free of Ca²⁺ and Mg²⁺ cations.

In some embodiments of the compositions and methods of the disclosure the pharmaceutical composition comprises about 1 µM to about 100 µM of an alpha-PKC inhibitor peptide.

In some embodiments of the compositions and methods of the disclosure the pharmaceutical composition comprises 1 µM of an alpha-PKC inhibitor peptide.

Another aspect of the disclosure is a method for decreasing inflammation at the site of a skin wound on an animal comprising the steps of providing a pharmaceutical composition comprising an alpha-PKC inhibitor and a pharmaceutically acceptable carrier that is free of Ca²⁺ and Mg²⁺ cations; and administering to a skin wound on an animal an effective amount of the pharmaceutical composition; whereby inflammation at the site of the skin wound is decreased.

### EXAMPLES

### MATERIALS AND EXPERIMENTAL METHODS

**Materials:** Tissue culture media and serum were purchased from Biological Industries (Beit HaEmek, Israel). Enhanced Chemical Luminescence (ECL) was performed with a kit purchased from BioRad (Israel). Monoclonal anti p-tyr antibody was purchased from Upstate Biotechnology Inc. (Lake Placid, NY, USA). Polyclonal and monoclonal antibodies to PKC isoforms were purchased from Santa Cruz (California, USA) and Transduction Laboratories (Lexington, KY). Horseradish peroxidase-anti-rabbit and anti-mouse IgG were obtained from Bio-Rad (Israel). Leupeptin, aprotinin, PMSF, DTT, Na-orthovanadate, and pepstatin were purchased from Sigma Chemicals (St. Louis, MO). Insulin (humulinR-recombinant human insulin) was purchased from Eli Lilly France SA (Fergersheim, France). IGF1 was purchased from Cytolab (Rehovot, Israel). Keratin 14 antibody was purchased from Babco-Convance (Richmond, CA) BDGF-BB was purchased from R&D systems (Minneapolis) and PKCα pseudosubstrate myristolated was purchased from Calbiochem (San Diego, CA). The Rapid cell proliferation Kit was purchased from Calbiochem (San Diego, CA).

The insulin analogs used were insulin lispro (HUMALOG®, Eli Lilly), insulin aspart (NOVOLOG®, Novo Nordisk), insulin glargine (LANTUS®, Sanofi Aventis), and recombinant regular human insulin (HUMULIN® R, Eli Lilly). Additional insulin analogs used were murine visfatin (ALEXIS Corporation, Lausen, Switzerland, Product Number ALX-201-318-C050) and L-α-Phosphatidylinositol-3,4,5-trisphosphate, Dipalmitoyl-, Heptaammonium Salt (Calbiochem; Cat. No. 524615) (L-alpha).

The Keratin 1 specific antibodies and western blotting secondary antibodies are commercially available.

**Isolation and culture of murine koratinocytes:** Primary keratinocytes were isolated from newborn skin as previously described. Keratinocytes were cultured in Eagle's Minimal Essential Medium (EMEM) containing 8% Chelex (Chelex-100, BioRad) treated fetal bovine serum. To maintain a proliferative basal cell phenotype, the final Ca²⁺ concentration was adjusted to 0.05 mM. Experiments were performed five to seven days after plating.

Medium A and B are both EMEM eagle's minimal essential medium from Biological Industries (Israel) containing 8% CHELEX™ treated fetal bovine serum. CHELEX™ is a strong chelator which binds free Ca²⁺ and Mg²⁺ ions to prevent these ions from being bioavailable to the cultured cells. Medium A does not contain KCl, Medium B contains KCI 0.4 mg/ml.

**Preparation of cell lysates for immunoprecipitation:** Culture dishes containing keratinocytes were washed with Ca²⁺/Mg²⁺-free PBS. Cells were mechanically detached and lysed in RIPA buffer (50 mM Tris·HCl pH 7.4; 150 mM NaCl; 1 mM EDTA; 10 mM NaF; 1% Triton x100; 0.1% SDS, 1% Na deoxycholate) containing a cocktail of protease and phosphatase inhibitors (20 µg/ml leupeptin; 10 µg/ml aprotinin; 0.1 mM PMSF; 1 mM DTT; 200 µM orthovanadate; 2 µg/ml pepstatin). The preparation was centrifuged in a microcentrifuge at maximal speed for 20 minutes at 4°C. The supernatant was used for immunoprecipitation.

**Immunoprecipitation:** The lysate was precleared by mixing 300 µg of cell lysate with 25 µl of Protein A/G Sepharose (Santa Cruz, CA, USA), and the suspension was rotated continuously for 30 minutes at 4°C. The preparation was then centrifuged at maximal speed at 4 °C for 10 minutes, and 30 µl of A/G Sepharose was added to the supernatant along with specific polyclonal or monoclonal antibodies to the individual antigens (dilution 1:100). The samples were rotated overnight at 4°C. The suspension was then centrifuged at maximal speed for 10 minutes at 4°C, and the pellet was washed with RIPA buffer. The suspension was again centrifuged at 15,000 x g (4°C for 10 minutes) and washed four times in TBST. Sample buffer (0.5 M Tris·HCl pH 6.8; 10% SDS; 10% glycerol; 4% 2-beta-mercaptoethanol; 0.05% bromophenol blue) was added and the samples were boiled for 5 minutes and then subjected to SDS-PAGE.

**Adenovirus constructs:** The recombinant adenovirus vectors were constructed as previously described by Saito et al. 54 J. Virol. 711 (1985).

**Transduction of keratinocytes with PKC isoform genes:** The culture medium was aspirated and keratinocyte cultures were infected with PKC recombinant adenoviruses encoding specific PKC isoforms such as PKCα for one hour. The cultures were then washed twice with MEM and re-fed. Ten hours post-infection cells were transferred to serum-free low Ca²⁺-containing MEM for 24 hours.

**PKC activity:** Specific PKC activity was determined in freshly prepared immunoprecipitates from keratinocyte cultures following appropriate treatments. These lysates were prepared in RIPA buffer without NaF. Activity was measured using the SignaTECT Protein Kinase C Assay System (Promega, Madison, WI, USA) according to the manufacturer's instructions. PKCα pseudosubstrate was used as the substrate in these studies.

**Cell proliferation:** Cell proliferation was measured by [³H]thymidine incorporation in 6 well plates. Cells were pulsed with [³H]thymidine (3 µCi/ml) for 1 h. After incubation, cells were washed five times with PBS and 5% TCA was added into each well for 1 h. The solution was removed and cells were solubilized in 1 M NaOH. The labeled thymidine incorporated into cells was counted in a ³H-window of TRI-CARB™ liquid scintillation counter.

**PKC immunokinase assay:** Purified and standardized PKC isozymes were kindly supplied by Dr. P. Blumberg (NCI, NIH, U.S.) and Dr. Marcello G. Kazanietz (University of Pennsylvania, School of Medicine). Primary keratinocytes were harvested in 500 µl 1% Triton Lysis Buffer (1% Triton-X 100, 10 µg/ml aprotinin and leupeptin, 2 µg/ml pepstatin, 1 mM PMSF, 1 mM EDTA, 200 µM Na₂VO₄, 10 mM NaF in 1 x PBS). Lysates were incubated at 4° C for 30 minutes, and spun at 16,000 x g for 30 minutes at 4 °C. Supernatants were transferred to a fresh tube. Immunoprecipitation of cell lysates was carried out overnight at 4 °C with 5 µg/sample anti-α6/GoH3 (PharMingen) and 30 µl/sample of protein A/G-Plus agarose slurry (Santa Cruz). Beads were washed once with RIPA buffer and twice with 50 mM Tris/HCl pH 7.5. 35 µl of reaction buffer (1 mM CaCl₂, 20 mM MgCl₂, 50 mM Tris·HCl pH 7.5) was added to each assay. To each assay, 5.5 µl/assay of a suspension of phospholipid vesicles containing either DMSO or 10 mM TPA was added to the slurry together with a standardized amount of specific PKC isozyme. The reaction was initiated by adding 10 µl/assay 125 mM ATP (1.25 µCi/assay [γ-32P] ATP, Amersham) and allowed to continue for 10 minutes at 30 °C. The beads were then washed twice with RIPA buffer. 30 µl/sample protein loading dye (3 x Laemmli, 5% SDS) was added and the samples were boiled for 5 minutes in a water bath. Proteins were separated by SDS-PAGE on a 8.5% gel, transferred onto Protran membranes (Schleicher & Schuell) and visualized by autoradiography. Phosphorylation of histones and phosphorylation of PKC substrate peptide was used as controls for PKC activity.

***In vivo* incision wound generation and inducement of inflammation:** Full thickness (20mm long) skin incisions were performed on the upper back of anesthetized C57BL/6J mice (6 mice per group).

**Other techniques:** Other techniques such as western blotting and the like were performed using standard protocols well known in the art such as those described in Sambrook et al., Molecular Cloning: A Laboratory Manual (3d ed. 2001).

In the examples and figures, the PKCα inhibitor was the myristolated peptide shown in SEQ ID NO: 1 unless otherwise specified. Similarly, the insulin was human recombinant insulin and is identified as "insulin," "USP insulin," or "Ins USP" unless otherwise specified.

### EXAMPLE 1

The following experiment was conducted to determine the efficacy of wound healing in *vitro* utilizing Insulin (10⁻⁶ M; 0.1 unit/ml) and PKCα inhibitor (Myr-pseudosubstrate PKCα peptide, 1 µM) prepared in various formulations.

First, murine keratinocytes were isolated and cultured. Briefly, primary keratinocytes were isolated from newborn skin in accordance with Alt *et al.* 2004; Li *et al.* 1996. Keratinocytes were cultured in Eagle's Minimal Essential Medium (EMEM) containing 8% Chelex (Chelex-100, BioRad) treated fetal bovine serum. To maintain a proliferative basal cell phenotype, the final Ca²⁺ concentration in the culture medium was adjusted to 0.05 mM.

After 5 days, confluent keratinocytes were subjected to *in vitro* scratch assays and wound healing was followed. Following wound formation, insulin + PKCα inhibitor were added to cell cultures in various formulations: Formulation A Dulbecco's Phosphate-Buffered Saline (DPBS-); Formulation B Phosphate-Buffered Saline (PBS) contained phosphates, potassium, calcium and magnesium; Formulation C Tris hydroxymethylaminoethane (CAS No.[77-86-1]) and formulation D contained Tris hydroxymethylaminoethane (CAS No.[77-86-1]) and KCl 0.4 mg/ml. Formulations were provided at a pH of approximately 7.2 and can comprise other components such as salts and the like necessary to maintain a given osmotic pressure.

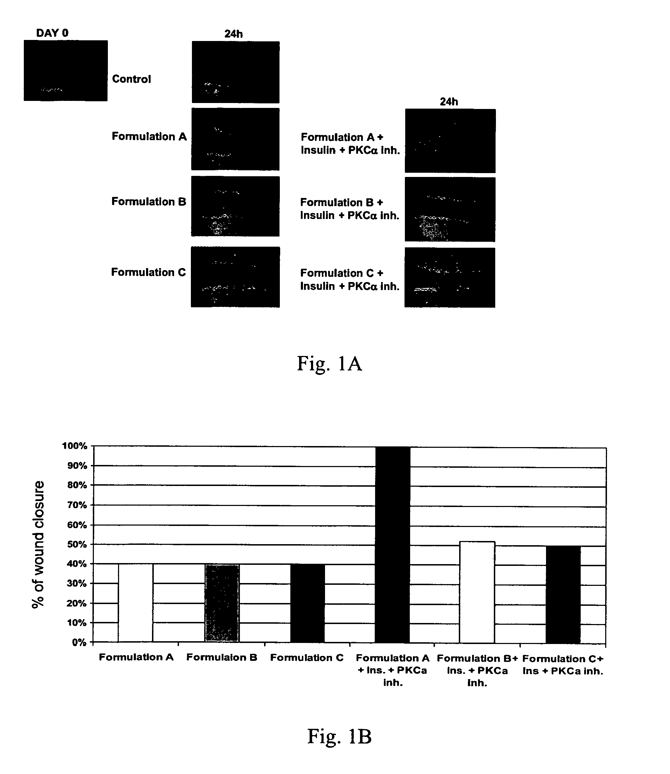

Wound closure was followed. Twenty-four hours following treatment, only cultures treated with Insulin + PKCα inhibitor in Formulation A showed closure of the wound as compared to non-treated control. This experiment was carried out in triplicate. Representative cell culture dishes are shown in Figure 1A. Figure 1B shows wound closure as percent of closure following 24 hours of treatment (p<0.05).

### EXAMPLE 2

The following experiment was conducted to further evaluate wound closure mediated by Insulin and PKCα inhibitor prepared in various formulations.

Full thickness (20mm long) skin incisions were performed on the upper back of anesthetized C57BL/6J mice (6 mice per group). Following the incision, wounds were treated daily with insulin (10⁻⁶ M; 0.1 units/ml); pseudosubstrate PKCα peptide, 1µM (PKCα inhibitor); or Insulin + PKCα inhibitor (Myr-pseudosubstrate PKCα peptide, 1µM and insulin 0.1 units) applied directly on the wounds in the various formulations (Formulations A-C) as described above.

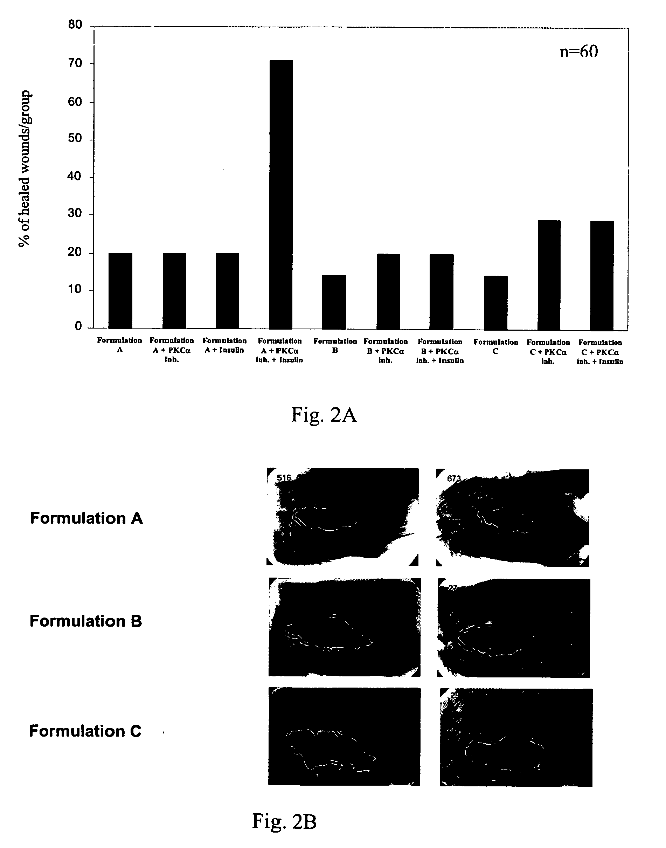

After 7 days, wounds were excised and the percentage of healed wounds was evaluated by examining the morphology and histology of the wounds. Results are presented as percent of healed wounds relative to the total number of wounds per group. Complete healing of wounds was dramatically induced by treatment of Insulin + PKCα inhibitor applied in Formulation A in comparison to marginal closure of wounds in Formulation B and Formulation C. For all formulations, treatments with insulin or pseudosubstrate peptide alone did not promote wound healing relative to control groups treated only with the formulations alone. The results are shown in Figure 2A. Representative photos of the wounds after 7 days of treatment are provided in Figure 2B.

### EXAMPLE 3

The following experiment was conducted to evaluate the anti-inflammatory effect of the pseudosubstrate PKCα peptide (PKCα inhibitor).

Full thickness (20mm long) skin incisions were performed on the upper back of anesthetized C57BL/6J mice (6 mice per group). Following incisions, wounds were treated daily with Myr-pseudosubstrate PKCα peptide, 1 µM applied directly on the wounds in the various formulations (Formulation A-C) described above.

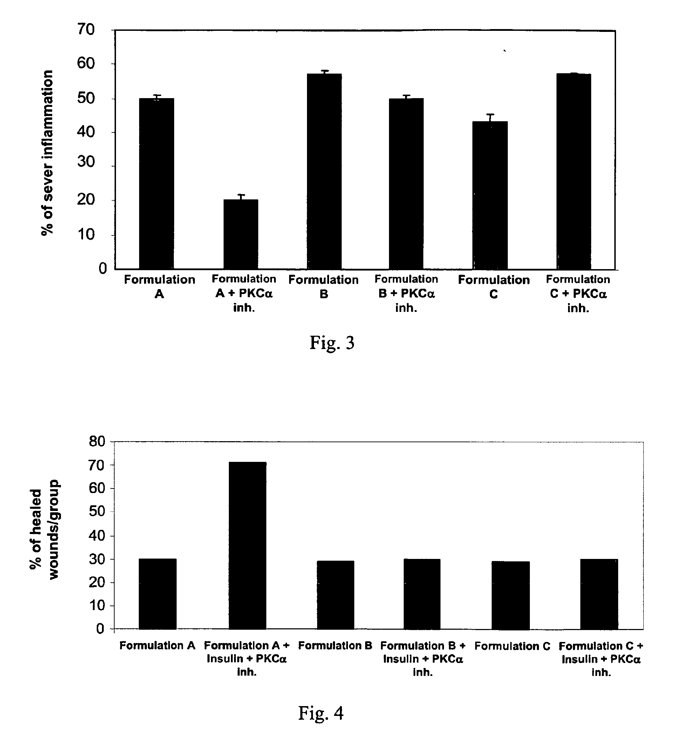

After 7 days, wounds were excised and subjected to histology and immunohistochemistry. Inflammatory burden was considered severe when at least 2 of the 3 following parameters were present at the wound gap: (1) Abscess formation at the wounded area, (2) excessive leukocytosis (>100 cells in a fixed field x200), (3) high WBC/RBC ratio in blood vessels where >20% of WBC content within the blood vessels is shown in a fixed field (x200). Results are summarized and presented as percent of wounds with severe inflammation relative to the number of wounds in the group. As seen in Figure 3, only when the pseudosubstrate PKCα peptide was applied in Formulation A was a significant reduction in severe inflammation noticed. No reduction in inflammatory burden was seen when treatments were applied in Formulation B or Formulation C.

### EXAMPLE 4

The following experiment was conducted to evaluate the effect of the pharmaceutical composition on granular tissue formation.

Full thickness (20mm long) skin incisions were performed on the upper back of anesthetized C57BL/6J mice (6 mice per group). Following incision, wounds were treated daily with Myr-pseudosubstrate PKCα peptide, 1 µM and insulin 0.1 unit/ml applied directly on the wounds in the various formulations (Formulation A-C) described above.

After 7 days, wounds were excised, fixed and assessed histologically following H&E staining, according to standard methods. Granulation tissue formation was assessed utilizing H&E staining and scored according to the percent of formed granulation tissue of the total wound area at the wound bed. When treated with Insulin + PKCα inhibitor, only wounds which were treated daily with Insulin + PKCα inhibitor in Formulation A showed significant increases in granulation tissue formation as compared to control and the Formulation B and Formulation C treated groups. Results are shown in Figure 4.

### EXAMPLE 5

The following experiment was conducted to determine if the content of the formulations affects the ability of pseudosubstrate PKCα peptide to inhibit PKCα activity.

Murine keratinocytes were isolated and cultured as described above. After five days, confluent keratinocytes were infected with PKCα recombinant adenovirus. Recombinant adenovirus vectors were constructed as described in Alt *et al.* 2001; Alt *et al.* 2004; Gartsbein *et al.* 2006. Keratinocyte cultures were infected with the supernatants containing PKC recombinant adenoviruses for one hour. The cultures were then washed twice with MEM and re-fed. Ten hours post-infection cells were transferred to serum-free low Ca²⁺-containing MEM for 24 hours.

Twenty-four hours following infection, cell were treated with PKCα inhibitor (Myr-pseudosubstrate PKCα peptide, I µM) for 15 minutes in various formulations (Formulation A and B) as described above.

The cell extracts were then subjected to PKC activity assay. First, primary keratinocytes were harvested in 500µl of 1% Triton Lysis Buffer (1% Triton-X 100, 10 µg/ml aprotinin and leupeptin, 2 µg/ml pepstatin, 1 mM PMSF, 1 mM EDTA, 200 µM Na₂VO₄, 10 mM NaF in 1 x PBS). Lysates were then incubated at 4°C for 30 minutes, and spun at 16,000 x g for 30 minutes at 4°C. Supernatants were transferred to a fresh tube. Immunoprecipitation of cell lysates was carried out overnight at 4°C with 5 µg/sample of anti-α6/GoH3 (PharMingen) antibody and a 30 µl/sample of protein A/G-Plus agarose slurry (Santa Cruz). Beads were washed once with RIPA buffer and twice with 50 mM Tris/HCl pH 7.5. 35 µl of reaction buffer (1 mM CaCl₂, 20 mM

MgCl₂, 50 mM Tris·HCl pH 7.5) was added to each assay. To each assay, 5.5 µl/assay of a suspension of phospholipid vesicles containing either DMSO or 10 mM TPA was added to the slurry together with a standardized amount of specific PKC isozyme. The reaction was initiated by adding 10 µl/assay 125 mM ATP (1.25 µCi/assay [γ-32P] ATP, Amersham) and allowed to continue for 10 minutes at 30° C. The beads were then washed twice with RIPA buffer. 30 µl/sample protein loading dye (3 x Laemmli, 5% SDS) was then added and the samples were boiled for 5 minutes in a water bath. Proteins were then separated by SDS-PAGE on an 8.5% gel, transferred onto Protran membranes (Schleicher & Schuell) and visualized by autoradiography. Phosphorylation of histones and phosphorylation of PKC substrate peptides were used as positive controls for PKC activity.

Specific PKC activity was measured with the use of the SignaTECT Protein Kinase C Assay System (Promega, Madison, WI, USA) according to the manufacturer's instructions. PKCα pseudosubstrate was used as the substrate in these studies.

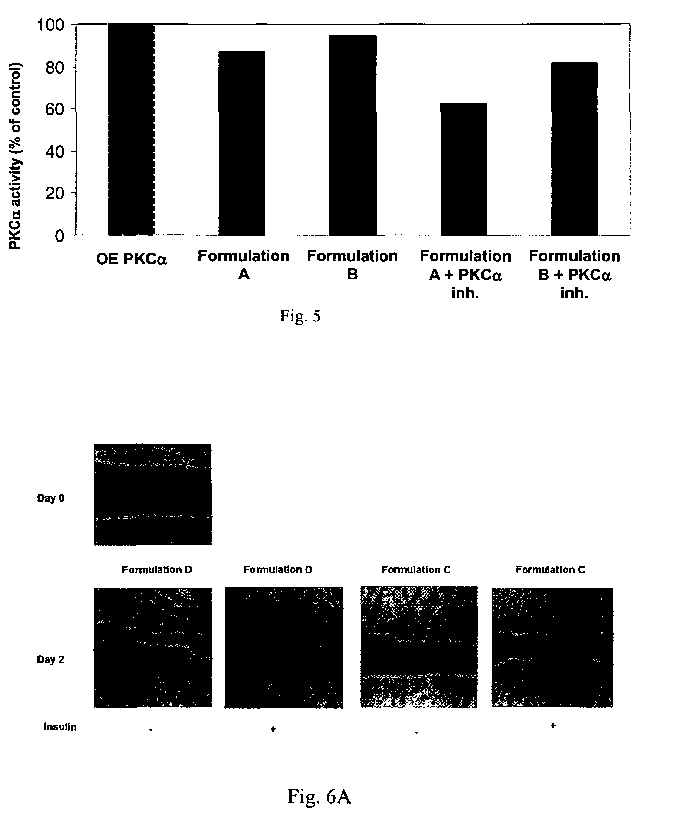

Only PKCα inhibitor in Formulation A was able to significantly inhibit PKCα activity in overexpressing cells relative to control formulations and untreated cell culture plates. Experiments were carried out in duplicate. Results are presented in Figure 5 as the percent reduction in PKCα activity relative to PKCα activity in control cells overexpressing PKCα.

### EXAMPLE 6

Further experiments were conducted to evaluate *in vitro* wound closure and cell proliferation mediated by insulin in various formulations.

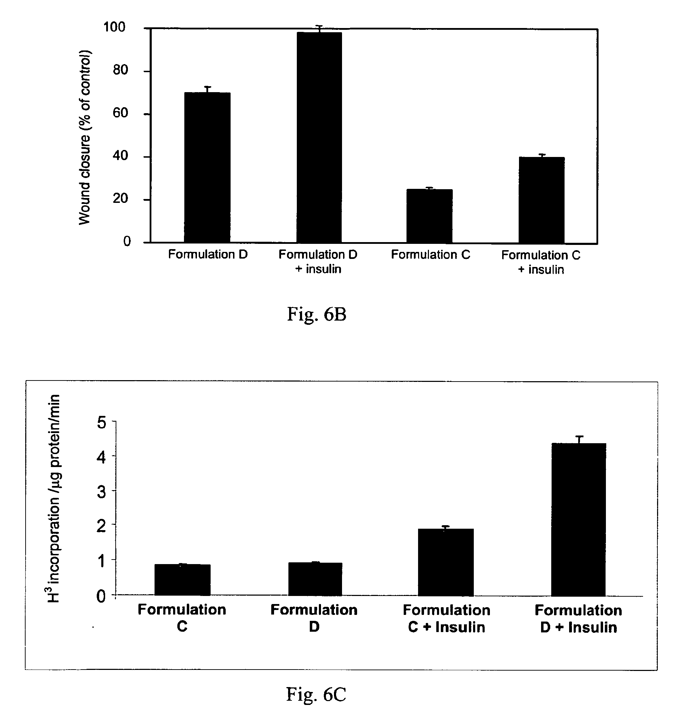

Murine keratinocytes were isolated and cultured as described above. After five days, confluent keratinocytes were subjected *in vitro* scratch assays to follow wound healing. Following wound formation Insulin (insulin 10⁻⁶ M; 0.1 units/ml) was added to the cell cultures in the various formulations (Formulation C and D) described above. Wound closure was followed for 48 hours. This experiment was carried out in triplicate. Representative cell culture dishes are shown in Figure 6A. Wound closure is presented as the percent of closure following 48 hours of treatment in Figure 6B.

Next, proliferation of cultured cells in the wound was evaluated utilizing thymidine incorporation (Figure 6C). Cell proliferation was measured by [³H]thymidine incorporation in 6 well plates. Cells were placed in 24 well plates and pulsed with [³H]thymidine (3 µCi/ml) for 1 h. After incubation, cells were washed five times with PBS and 5% TCA was added to each well for 1 h. This solution was then removed and cells were solubilized in 1 M NaOH. The labeled thymidine incorporated into the cells was counted in the ³H-window of a liquid scintillation counter. As shown in Figures 6A-C the addition of KCl changed insulin induced wound closure and cell proliferation.

### EXAMPLE 7

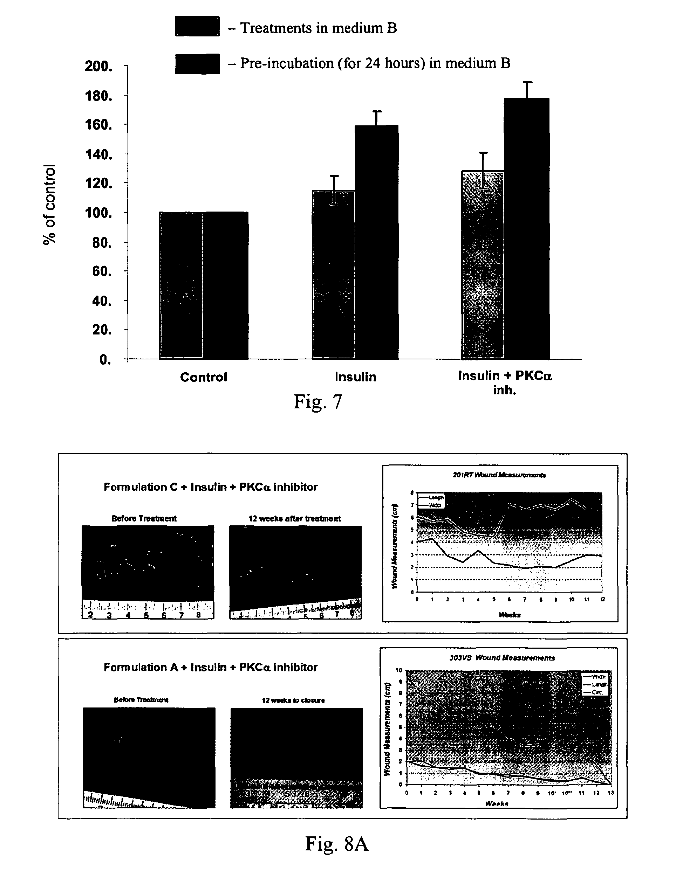

The influence of pre-incubation of keratinocytes in medium B on the effects of Insulin and Insulin + PKCα inhibitor on cell proliferation *in vitro* was evaluated. Cultures of 5 day old, confluent keratinocytes from the tails of adult mice (7-10 months up to 2 years) were allowed to proliferate *in vitro.* After 5 days in MEM (medium A), the growth medium was changed to medium B (described above). Parallel to the medium changing or 24 hours ,following it, cells were treated with Insulin or with Insulin + PKCα inhibitor. The proliferation rate of the cells was measured with the commercially available Rapid Cell Proliferation Kit (Cat No. QIA127; Calbiochem). The Experiment was carried out in hexaplicate. Results are presented as percent of untreated cells (control). As can be seen from Figure 7, pre-incubation of the cells in medium B for 24 hours enhances the effects of Insulin and Insulin + PKCα inhibitor on cell proliferation.

### EXAMPLE 8A

Human patients with chronic foot ulcers were treated daily by topical application of Insulin + PKCα inhibitor applied in Formulation A (results shown in lower panel of Figure 8A) or in Formulation C (results shown in upper panel of Figure 8A) for a period of 12 weeks. While Insulin + PKCα inhibitor applied in Formulation A showed full closure by 12 weeks, no significant healing was seen in the ulcers of patients treated with Insulin + PKCα inhibitor in Formulation C. Patients wounds were followed weekly and measured utilizing VISITRAK® (Smith & Nephew). Follow-up graphs of wound width and wound length are presented for a 12 weekly measurements of both patients (lower panels).

### EXAMPLE 8B

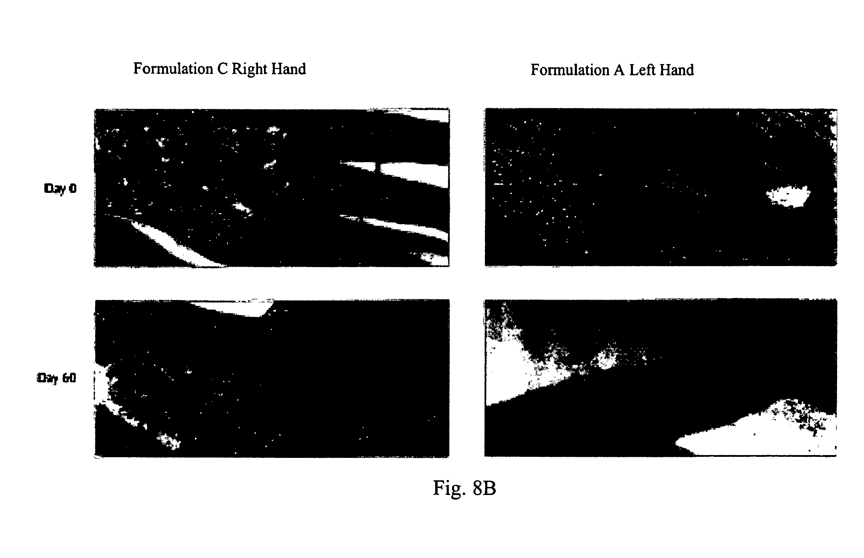

A human patient suffering from diabetic wounds was treated daily by topical application of Insulin + PKCα inhibitor applied in Formulation A (results shown in left panel of Figure 8B) or in Formulation C (results shown in right panel of Figure 8B) for 60 days. While Insulin + PKCα inhibitor applied in Formulation A showed full wound closure and healing by 60 days, no significant healing was seen in the wounds treated with Insulin + PKCα inhibitor in Formulation C. Figure 8B shows follow-up documentation of wounds at day 0 and at 60 days.

### EXAMPLE 9

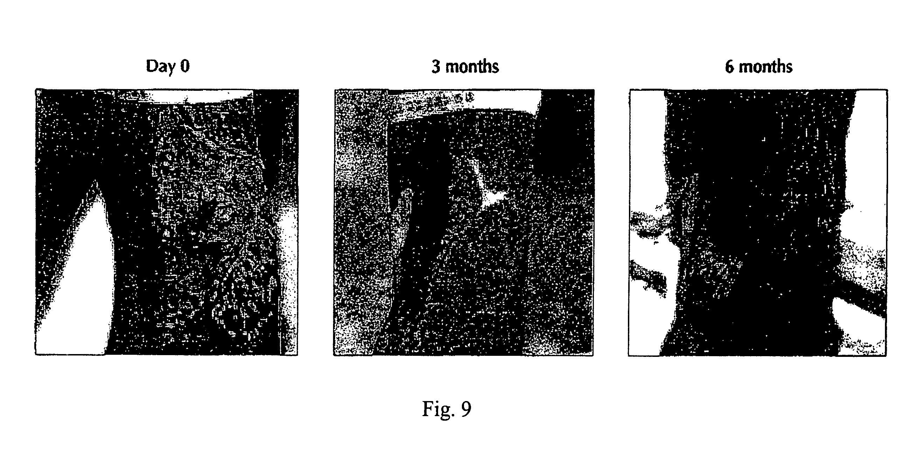

A one-year-old female quarter horse suffered from an exuberant granulation tissue (proud flesh) wound without healing for a period of months. The wound was treated daily with Insulin + PKCα inhibitor in Formulation A for 3 months. After this period of time, the wound was completely closed and healed. A follow-up at six months showed complete tissue regeneration. The results are shown in Figure 9.

### EXAMPLE 10

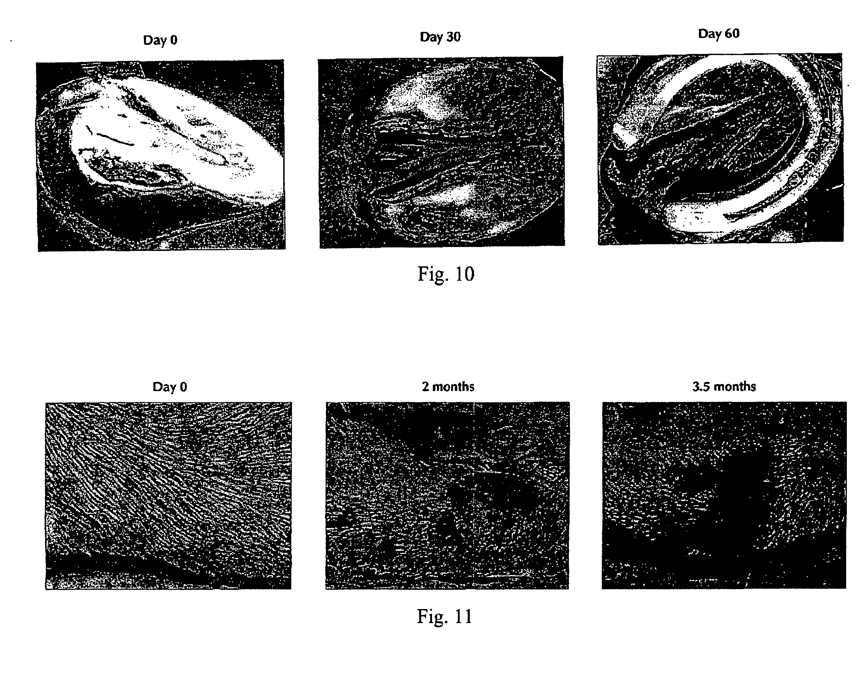

A two-year-old horse had a hoof wound diagnosed as a chronic solar abscess with osteomyelitis. No healing of this wound had occurred for a period of several months. Daily treatment with Insulin + PKCα inhibitor in Formulation A was performed by direct application of the composition to the wound for 30 minutes. As shown in Figure 10, within 1 month of treatment the wound size was significantly reduced and within 2 months the wound had completely closed and healed.

### EXAMPLE 11

A dog suffering wounds on its paws due to constant licking (*i.e.* acral lick) was treated using conventional treatments for a period of several months without any healing. Daily treatment with Insulin + PKCα inhibitor in Formulation A was performed and the wound was completely closed and healed within 2 months. After 3.5 months of treatment, complete fur re-growth was observed. The results are shown in Figure 11.

### EXAMPLE 12

Four insulin analogs prepared in Formulation A were studied to determine whether insulin analogs alone could promote wound healing. Full thickness (20mm long) skin incisions were performed on the upper back of anesthetized C57BL/6J mice (6 mice per group). Following incision, wounds were treated daily with 0.1 unit/ml of various insulin analogs in Formulation A (described above) placed directly on the wounds. The insulin analogs studied were insulin lispro (HumL), insulin aspart (Novo), insulin glargine (LANTUS®), and HUMULIN® R (HumR). After 7 days, wounds were excised, fixed and assessed histologically following H&E staining.

Percent wound healing was separately assessed by measuring epidermal basal layer formation and granulation tissue formation. Epidermal closure was assessed by utilizing keratin 14 staining to detect epidermal basal layer formation. Wounds that exhibited complete epidermal reconstruction were considered healed. Granulation tissue formation was assessed utilizing H&E staining and scored according to the percent of formed granulation tissue in the total wound area at the wound bed. Wounds that exhibited >70% formation of granulation tissue were considered healed.

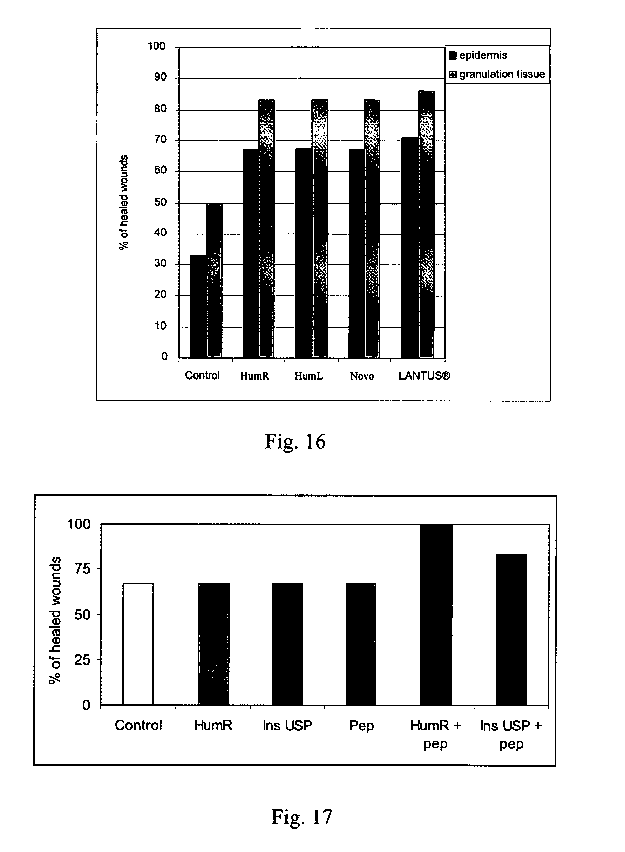

The results demonstrate that the insulin analogs alone in Formulation A increase wound healing and wound closure relative to controls. The results are shown in Figure 16. In Figure 16 the insulin analogs are referred to by abbreviations of trademark names: "HumL" for insulin lispro, "Novo" for insulin aspart, "LANTUS®" for insulin glargine, and "HumR" for HUMULIN® R.

### EXAMPLE 13

Wound healing was measured by assessing formation of granulation tissue after treatment with regular recombinant human insulin, and USP insulin PKCα pseudosubstrate inhibiting peptide as indicated in Figure 17 to identify synergistic effects.

Full thickness (20mm long) skin incisions were performed on the upper back of anesthetized C57BL/6J mice (6 mice per group). Following incision, wounds were treated daily with PKCα pseudosubstrate inhibiting peptide (1 µg/ml) or with 0.1 unit/ml of regular recombinant human insulin, USP insulin, and PKCα pseudosubstrate inhibiting peptide (1 µg/ml) in Formulation A as indicated in Figure 17 and placed directly on the wounds. After 7 days, wounds were excised, fixed and assessed histologically following H&E staining.

Granulation tissue formation was assessed using H&E staining and scored according to the percent of formed granulation tissue in the total wound area of the wound bed. Wounds that exhibited >70% formation of granulation tissue were considered healed.

When compared to control wounds or to each compound administered alone, the results demonstrate that USP insulin combined with PKCα pseudosubstrate inhibiting peptide results in synergistic effects on wound healing similar to regular recombinant human insulin + PKCα pseudosubstrate inhibiting peptide. This data indicates that the combination of insulin analogs and PKCα pseudosubstrate inhibiting peptide may be helpful in promoting the formation of granulation tissue and treating wounds.

The results are shown in Figure 17. In Figure 17, regular recombinant human insulin and USP insulin are referred to by the abbreviations "HumR" and "Ins USP," respectively. PKCα pseudosubstrate inhibiting peptide is referred to as "pep."

### EXAMPLE 14

Inflammation after treatment with insulin analogs, recombinant human insulin and PKCα pseudosubstrate inhibiting peptide was measured to determine the effects of these treatments on inflammation.

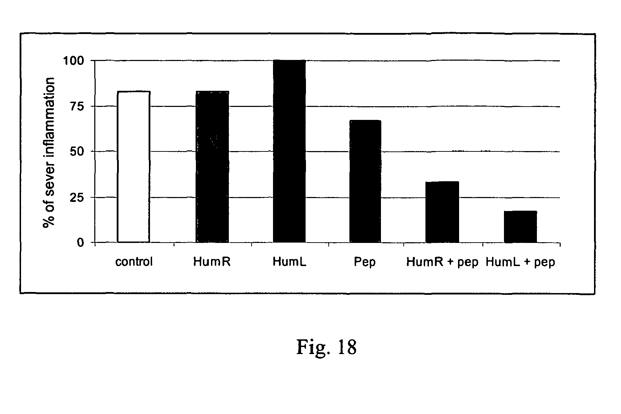

The level of severe inflammation was measured at skin wound sites on C57BL/6J mice (6 mice per group). Wounds were prepared by incision as described above. Daily treatment was performed with PKCα pseudosubstrate inhibiting peptide (1 µg/ml), 0.1 unit/ml of recombinant human insulin, or 0.1 unit/ml insulin lispro in Formulation A as indicated in Figure 18. An emulsion was prepared using standard methods and was delivered to the skin with a gauze bandage which functioned as a drug eluting scaffold. After 7 days, skin tissues were excised, fixed and assessed histologically following H&E staining.

Severe inflammation was assessed utilizing the following parameters (as described above):
(1) Abscess formation
(2) Excessive leukocytosis (>100 cells in a fixed field x200)
(3) High WBC/RBC ratio in blood vessels where >20% of WBC content within the blood vessels is shown in a fixed field x200.

The total percent of severe inflammation was determined by consolidating the data recorded according to each of the above parameters observed for each specimen. Inflammatory burden was considered severe when at least 2 of the 3 above parameters were present at the wound gap.

The results demonstrate that the insulin analogs and recombinant human insulin synergistically promote reduction of the inflammatory response in severely inflamed skin when combined with PKCα inhibiting peptide in Formulation A relative to the controls. This data indicates that the treatments shown in Figure 18 can be used in treating inflammatory disorders of the skin, such as inflammation caused by inflammatory skin diseases. This data also indicates that emulsion formulations and drug eluting scaffolds such as gauze sponges can be used to deliver the pharmaceutical compositions disclosed herein.

The results are depicted in Figure 18. In Figure 18, regular recombinant human insulin and insulin lispro are identified as "HumR" and "HumL," respectively. PKCα pseudosubstrate inhibiting peptide is identified as "pep."

### EXAMPLE 15

The influence of incubation of keratinocytes in Medium A and Medium B and treatment with murine visfatin and L- α-Phosphatidylinositol-3,4,5-trisphosphate, Dipalmitoyl-, Heptaammonium Salt (L-alpha) (Calbiochem; Cat. No. 524615) on expression of keratin 1.

Primary skin keratinocytes isolated from the tails of adult mice (7-10 months up to 2 years) were maintained in medium A (MEM) as described above. After 5 days in (medium A), the growth medium in half of the cultured plates was replaced with medium B (as described above)

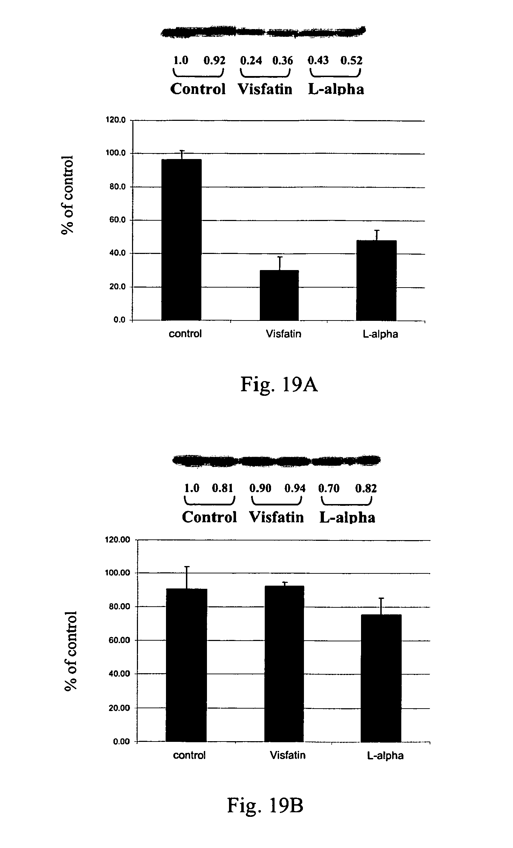

Next visfatin or L-alpha were each individually added to cells cultured in medium A (Figure 19A) and cells cultured in medium B (Figure 19B) as indicated in Figure 19. The final concentration in the culture medium of visfatin was 0.0001 µg/ml visfatin. The final concentration in the culture medium of L-alpha was 100 ng/ml.

Cell differentiation was induced by elevating calcium from 0.05 mM to 0.12 mM as described above. Twenty-four (24) hours after differentiation cells were harvested and Western Blot analysis was performed. An antibody specific for keratin 1 was used to assess the expression of the keratin 1 protein using standard Western blotting techniques. Keratin 1 expression was then quantified using standard densitometric methods

Keratin 1 is a spinous differentiation marker. The expression of keratin 1 in keratinocytes is associated with the loss of mitotic activity in epidermal keratinocytes and restricted to an intermediate stage of terminal differentiation. Reduced keratinocyte differentiation is associated with keratinocyte migration and proliferation, and thus epidermal formation.

The results indicate that the expression of keratin 1 decreased relative to the control sample after treatment with both visfatin and L-alpha in medium A. (Fig. 19A) In contrast, the keratin 1 expression of keratinocytes cultured in medium B was not significantly altered by treatment of either visfatin or L-alpha. (Fig. 19B) Taken together, these results indicate that insulin analogs such as visfatin or L-alpha can inhibit keratinocyte differentiation and promote epidermis formation when provided in medium A.

### DETAILED DESCRIPTION OF THE FIGURES

### FIGURE 1

### Efficacy of wound healing in vitro utilizing Insulin + PKCα inhibitor prepared in various formulations.

Cultures of 5 day old, confluent keratinocytes were subjected to *in vitro* scratch assays and wound healing was examined.

Following wound formation, Insulin and PKCα inhibitor (insulin 10⁻⁶ M; 0.1 units/ml), Myr-pseudosubstrate PKCα peptide, 1 µM) were added to cell cultures in various formulations (Formulation A-C) described above and wound closure was followed. Twenty-four (24) hours following treatment, only cells treated with Insulin and the PKCα inhibitor provided in Formulation A showed closure of the wound relative to untreated controls. This experiment was carried out in triplicate. Figure 1A shows photographs of representative cell culture plates. Figure 1B shows the percentage of wound closure following 24 hours of treatment (p<0.05).

### FIGURE 2

### Insulin + PKCα inhibitor promote significant wound closure only in Formulation A.

Full thickness (20mm long) skin incisions were performed on the upper back of anesthetized C57BL/6J mice (6 mice per group). Following incision, wounds were treated daily with Insulin (10⁻⁶ M; 0.1 units/ml), PKCα inhibitor (Pseudosubstrate PKCα peptide, 1 µM) or Insulin + PKCα inhibitor (Pseudosubstrate PKCα peptide, 1 µM and insulin 0.1 units) applied directly to the wounds in the various formulations (Formulation A-C) described above. After 7 days, wounds were excised and the percentage of healed wounds was evaluated by examining the morphology and histology of the wounds. In Figure 2A, results are presented as percent of healed wounds per total of wounds per group. Complete healing and closure of wounds was induced by treatment of Insulin + PKCα inhibitors applied in Formulation A. In contrast, only marginal closure of wounds was observed with Formulation B and Formulation C. For all formulations conditions, the treatment with insulin or pseudosubstrate peptide alone did not promote wound healing efficacy as compared to control groups treated only with the various formulations. Figure 2B shows photographs from representative wound biopsies.

### FIGURE 3

### PKCα inhibitor reduces the severe inflammatory burden at the wound bed only when administered in Formulation A.

Full thickness (20mm long) skin incisions were performed on the upper back of anesthetized C57BL/6J mice (6 mice per group). Following incision, wounds were treated daily with PKCα inhibitor (Pseudosubstrate PKCα peptide, 1 µM) applied directly to the wounds in the various formulations (Formulation A-C) described above. After 7 days, wounds were excised and subjected to histology and immunohistochemistry. Inflammatory burden was considered severe when at least 2 of the 3 following parameters were present at the wound gap: (1) Abscess formation at the wounded area, (2) excessive leukocytosis (>100 cells in a fixed field x200), (3) high WBC/RBC ratio in blood vessels where >20% of WBC content within the blood vessels is shown in a fixed field (x200). Results are summarized and presented as percent of wounds with severe inflammation per total wounds in the group. As seen in the bar graph, only when PKCα inhibitor was applied in Formulation A was a significant reduction in severe inflammation observed. No reduction in inflammatory burden was seen when treatments were applied in Formulation B or Formulation C.

### FIGURE 4

### Insulin + PKCα inhibitor induce granulation tissue formation when treated in Formulation A.

Full thickness (20mm long) skin incisions were performed on the upper back of anesthetized C57BL/6J mice (6 mice per group). Following incision, wounds were treated daily with Insulin and PKCα inhibitor (Pseudosubstrate PKCα peptide, 1 µM and insulin 0.1 unit/ml) applied directly on the wounds in various formulations (Formulation A-C) as described above. After 7 days, wounds were excised, fixed and assessed histologically following H&E staining. Granulation tissue formation was assessed utilizing H&E staining and scored according to the percent of granulation tissue formed relative to the total wound area at the wound bed. Only wounds which were treated daily with Insulin and PKCα inhibitor in Formulation A showed a significant increase in granulation tissue formation as compared to control, Formulation B and Formulation C treated groups.

### FIGURE 5

### Formulation conditions affect the ability of pseudosubstrate PKCα peptide to inhibit PKCα activity.

Cultures of 5 day old, confluent keratinocytes were infected with a recombinant adenovirus encoding PKCα. Twenty-four (24) hours following infection, cells were treated with PKCα inhibitor (Myr-pseudosubstrate PKCα peptide, 1 µM) for 15 minutes in the formulations (Formulation A, B) as indicated in Figure 5. Following treatment, cells were lysed and subjected to PKCα activity assay as described above. Only PKCα inhibitor provided in Formulation A was able to significantly inhibit PKCα activity in overexpressing cells relative to controls. Experiments were carried out in duplicate. Results are presented as the percent reduction in PKCα activity relative to PKCα overexpressing control cells.

### FIGURE 6

### Efficacy of wound closure and cell proliferation in vitro utilizing insulin is dependent on formulation content.

Cultures of 5 day old, confluent keratinocytes were subjected to *in vitro* scratch assays and wound healing was examined. Following wound formation, Insulin (10⁻⁶ M; 0.1 units/ml) was added to cell cultures in the various formulations (Formulation C and D) as described above. Wound closure was followed for 48 hours. Experiments were carried out in triplicate. (A) Photographs of representative cell culture dishes are presented. (B) Wound closure is presented as percent of closure following 48 hours of treatment. (C) Proliferation assays were performed on cultured cells in the wound using the thymidine incorporation proliferation assay described above. Insulin by itself induced partial wound closure and cell proliferation when provided in Formulation D, which contains KCI. Formulation C inhibited insulin induced wound closure and cell proliferation as seen in Figures 6A-6C.

### FIGURE 7

### Pre-incubation in medium B enhances the effects of Insulin and Insulin + PKCα inhibitor on cell proliferation in vitro.

Cultures of 5 day old, confluent keratinocytes prepared from the tails of adult mice (7-10 months up to 2 years) were subjected to proliferation assays utilizing a commercially available Rapid Cell Proliferation Kit (Cat. No. QIA127; Calbiochem). After 5 days in MEM, the growth medium was changed to medium B as described above.

Parallel to the medium change or 24 hours following it, cells were treated with Insulin or with Insulin + PKCα inhibitor. Experiments were carried out in hexaplicate. Results are presented as percent of untreated cells (control). Pre-incubation of the cells in medium B for 24 hours enhances the effects of Insulin and Insulin + PKCα inhibitor on cell proliferation as shown in Figure 7.

### FIGURE 8

### Insulin + PKCα inhibitor prepared in Formulation A but not in Formulation C induces wound healing of chronic, non-healing wounds.

Patients with chronic diabetes associated ulcers, such as diabetes associated foot and hand ulcers, were treated daily by topical application of Insulin + PKCα inhibitor applied in Formulation A (Figure 8A, lower panel) or in Formulation C (Figure 8A, upper panel) for 12 weeks. Insulin + PKCα inhibitor applied in Formulation A showed full closure by 12 weeks, no significant healing was seen in the ulcers of patients treated with Insulin + PKCα inhibitor in formulation C. Patients wounds were followed weekly and measured utilizing VISITRAK® (Smith & Nephew). Follow-up graphs of wound width and wound length are presented for a 12 weekly measurements of both patients (right panels in Figure 8A).

A patient suffering from diabetic wounds was treated daily with topical application of Insulin + PKCα inhibitor applied in Formulation A (Figure 8B, right panel) or in Formulation C (Figure 8B, left panel) for 60 days. Insulin + PKCα inhibitor applied in Formulation A provided full healing and wound closure by 60 days. No significant healing was seen in wounds treated with Insulin + PKCα inhibitor in Formulation C. Follow-up photo-documentation of wounds at day 0 and at 60 days is presented in Figure 8B.

### FIGURE 9

### Insulin + PKCα inhibitor prepared in Formulation A induce healing of Proud Flesh chronic wounds in horses.

A one-year-old female quarter horse suffered from exuberant granulation tissue (proud flesh) wound, without healing for a period of months. The wound was treated daily with Insulin + PKCα inhibitor in Formulation A for 3 months. After this period of time the wound was completely closed and healed. In a follow up at six months, complete tissue regeneration was observed.

### FIGURE 10

### Insulin + PKCα inhibitors prepared in Formulation A heal a chronic solar abscesses and osteomyelitis.

A two year old horse had a hoof wound diagnosed as a chronic solar abscess with osteomyelitis without healing for a period of months. Daily treatment with Insulin + PKCα inhibitor in Formulation A was performed by direct application of the composition to the wound for 30 min. As shown in Fig. 10, within 1 month of treatment the wound size had been reduced significantly and within 2 months the wound had completely closed and healed.

### FIGURE 11

### Insulin + PKCa inhibitor prepared in Formulation A heal chronic wounds caused by self trauma (acral lick).

A dog suffering chronic acral lick wounds on its paws due to constant self-licking was treated using conventional methods for several months without healing. The wound was treated daily with topically applied Insulin + PKCα inhibitor in Formulation A. Within 2 months the wound had completely closed and healed. Within 3.5 months complete fur re-growth was observed.

### FIGURE 12

A schematic representation of insulin lispro (rDNA origin) known by the trademark HUMALOG®. The amino acid sequences of the alpha chain (SEQ ID NO: 57) and beta chain (SEQ ID NO: 58) of insulin lispro are each shown.

### FIGURE 13

A schematic representation of the primary structure of the human insulin analog insulin aspart (rDNA origin), known by the trademark NOVOLOG®. The amino acid sequences of the alpha chain (SEQ ID NO: 57) and beta chain (SEQ ID NO: 59) of insulin aspart are each shown.

### FIGURE 14

A schematic representation of the primary structure of the human insulin analog insulin glargine (rDNA origin) known by the trademark LANTUS®. The amino acid sequences of the alpha chain (SEQ ID NO: 60) and beta chain (SEQ ID NO: 61) of LANTUS® are each shown.

### FIGURE 15

A schematic representation of the primary structure of regular recombinant human insulin, known by the trademarks HUMULIN® R and NOVOLIN® R The amino acid sequences of the alpha chain (SEQ ID NO: 57) and beta chain (SEQ ID NO: 62) of HUMULIN® R are each shown.

### FIGURE 16

### Various Insulin Analogs similarly affect wound healing provided in Formulation A.

Full thickness (20mm long) skin incisions were performed on the upper back of anesthetized C57BL/6J mice (6 mice per group). Following incision, wounds were treated daily with 0.1 unit/ml of the insulin analogs indicated in Figure 6 in Formulation A (described above) placed directly on the wounds. The insulin analogs studied were insulin lispro ("HumL"), insulin aspart ("Novo"), insulin glargine ("LANTUS®"), and recombinant human insulin ("HumR"). After 7 days, wounds were excised, fixed and assessed histologically following H&E staining.

Percent wound healing was assessed by measuring epidermal basal layer formation and granulation tissue formation. Epidermal closure was assessed by utilizing keratin 14 staining to detect epidermal basal layer formation. Wounds that exhibited complete epidermal reconstruction were considered healed. Granulation tissue formation was assessed utilizing H&E staining and scored according to the percent of granulation tissue formed relative to the total wound area at the wound bed. Wounds that exhibited >70% formation of granulation tissue were considered healed.

The insulin analogs are identified by abbreviations of trademark names: "HumL" for insulin lispro, "Novo" for insulin aspart, "LANTUS®" for insulin glargine, and "HumR" for HUMULIN® R.

### FIGURE 17

### USP insulin combined with PKCα inhibiting peptide promotes wound healing similarly to HUMULIN® R + PKCα inhibiting peptide.

Full thickness (20mm long) skin incisions were performed on the upper back of anesthetized C57BL/6J mice (6 mice per group). Following incision, wounds were treated daily with PKCα pseudosubstrate inhibiting peptide (1 µg/ml) or with 0.1 unit/ml of HUMULIN® R or USP insulin in Formulation A (described above) was placed directly on the wounds. After 7 days, wounds were excised, fixed and assessed histologically following H&E staining.

Granulation tissue formation was assessed utilizing H&E staining and scored according to the percent of granulation tissue formed relative to the total wound area at the wound bed. Wounds that exhibited >70% formation of granulation tissue were considered healed.

Regular recombinant human insulin and USP insulin are identified by the abbreviations "HumR" and "Ins USP," respectively. PKCα pseudosubstrate inhibiting peptide is identified as "pep."

### FIGURE 18

### Insulin analogs combined with PKCα inhibiting peptide synergistically promote reduction of the inflammatory response in severely inflamed skin.

The level of severe inflammation was measured at the skin wound sites on C57BL/6J mice (6 mice per group). Wounds were prepared by incision as described above. Daily treatment was performed with PKCα pseudosubstrate inhibiting peptide (1 µg/ml) or with 0.1 unit/ml of HUMULIN® R, or insulin lispro in Formulation A (described above) as indicated in Figure 19. An emulsion was prepared and was placed on the skin by delivery from a gauze bandage functioning as a drug eluting scaffold. After 7 days, skin tissues were excised, fixed and assessed histologically following H&E staining.

Severe inflammation was assessed utilizing the following parameters:
(1) Abscess formation
(2) Excessive leukocytosis (>100 cells in a fixed field x200)
(3) High WBC/RBC ratio in blood vessels where >20% of WBC content within the blood vessels is shown in a fixed field x200.
Inflammatory burden was considered severe when at least 2 of the 3 above parameters were present at the wound gap.

The total percent of severe inflammation was determined by consolidating the data recorded according to each of the above parameters observed for each specimen.

HUMULIN® R and insulin lispro are identified by the abbreviations "HumR" and "HumL," respectively. PKCα pseudosubstrate inhibiting peptide is identified as "pep."

### FIGURE 19

### Expression of keratin 1 in old Keratinocyte treated with Visfatin and L-alpha in medium A and medium B.

Primary skin keratinocytes prepared from adult mouse tails (7-10 months up to 2 years) were maintained in medium A (MEM). After 5 days in medium A, the growth medium in half of the cultured plates was replaced with medium B (as described above). Visfatin or L-alpha were then provided to cells cultured in medium A and medium B.

Cell differentiation was then induced by elevating calcium levels in the culture medium from 0.05 mM to 0.12 mM. Twenty-four (24) hours after differentiation was induced cells were harvested and Western Blot analysis was performed. A commercially available keratin 1 specific antibody was then used to assess the expression of keratin 1 in the cellular lysates. Expression was assessed using standard Western blotting and densitometry techniques.

All references (*e.g.* journal articles, patent documents, and accession numbers) cited herein are incorporated by reference in their entirety.

### REFERENCES

Adams JC and Watt FM (1990) Changes in keratinocyte adhesion during terminal differentiation: reduction in fibronectin binding precedes alpha 5 beta 1 integrin loss from the cell surface. Cell 63: 425-435.
Alt A, Gartsbein M, Ohba M, Kuroki T, and Tennenbaum T (2004) Differential regulation of alpha6beta4 integrin by PKC isoforms in murine skin keratinocytes. Biochem Biophys Res Commun 314: 17-23.
Alt A, Ohba M, Li L, Gartsbein M, Belanger A, Denning MF, Kuroki T, Yuspa SH, and Tennenbaum T (2001) Protein kinase Cdelta-mediated phosphorylation of alpha6beta4 is associated with reduced integrin localization to the hemidesmosome and decreased keratinocytes attachment. Cancer Res 61: 4591-4598.
Ausubel FM, et al. (1989) Current Protocols in Molecular Biology, Wiley Interscience.
Azzi A, Boscoboinik D, and Hensey C (1992) The protein kinase C family. Eur J Biochem 208: 547-557.
Bell E, Sher S, Hull B, Merrill C, Rosen S, Chamson A, Asselineau D, Dubertret L, Coulomb B, Lapiere C, Nusgens B, and Neveux Y (1983) The reconstitution of living skin. J Invest Dermatol 81: 2s-10s.
Blumberg PM (1991) Complexities of the protein kinase C pathway. Mol Carcinog 4: 339-344. Boyce ST and Ham RG (1983) Calcium-regulated differentiation of normal human epidermal keratinocytes in chemically defined clonal culture and serum-free serial culture. J Invest Dermatol 81: 33s-44s.
Bradshaw D, Hill CH, Nixon JS, and Wilkinson SE (1993) Therapeutic potential of PKC inhibitors. Agents Actions 38: 135-147.
Breitkreutz D, Bohnert A, Herzmann E, Bowden PE, Boukamp P, and Fusenig NE (1984) Differentiation specific functions in cultured and transplanted mouse keratinocytes: environmental influences on ultrastructure and keratin expression. Differentiation 26: 154-169. Breitkreutz D, Stark HJ, Plein P, Baur M, and Fusenig NE (1993) Differential modulation of epidermal keratinization in immortalized (HaCaT) and tumorigenic human skin keratinocytes (HaCaT-ras) by retinoic acid and extracellular Ca2+. Differentiation 54: 201-217.
Cadena, Gill (1993) Protein Expression and Purification 4:177.
Castagna M, Takai Y, Kabuchi K, Kikkawa U, and Nishizuka Y (1982) Direct activation of calcium-activated phospholipid dependent protein kinase by tumor-promoting phorbol esters. J Biol Chem 257: 7847-7851.
Chakravarthy BR, Isaacs RJ, Morley P, Durkin JP, and Whitfield JF (1995) Stimulation of protein kinase C during Ca2+-induced keratinocyte differentiation. Selective blockade of MARCKS phosphorylation by calmodulin. J Biol Chem 270: 1362-1368.
Chauhan VPS, Chauha A, Deshmukh DS, and Brockerhoff H (1990) Lipid activators of protein kinase C. Life Sci 47: 981-986.
Coligan et al., Current Protocols in Immunology, Wiley Interscience, 1991, Unit 9.
De M, I and Theoret CL (2004) Spatial and temporal expression of types I and II receptors for transforming growth factor beta in normal equine skin and dermal wounds. Vet Surg 33: 70-76.
Denning MF, Dlugosz AA, Cheng C, Dempsey PJ, Coffey RJJ, Threadgill DW, Magnuson T, and Yuspa SH (2000) Cross-talk between epidermal growth factor receptor and protein kinase C during calcium-induced differentiation of keratinocytes. Exp Dermatol 9: 192-199.
Denning MF, Dlugosz AA, Williams EK, Szallasi Z, Blumberg PM, and Yuspa SH (1995) Specific protein kinase C isozymes mediate the induction of keratinocyte differentiation markers by calcium. Cell Growth Differ 6: 149-157.
Deucher A, Efimova T, and Eckert RL (2002) Calcium-dependent involucrin expression is inversely regulated by protein kinase C (PKC)alpha and PKCdelta. J Biol Chem 277: 17032-17040.
Diegelmann RF and Evans MC (2004) Wound healing: an overview of acute, fibrotic and delayed healing. Front Biosci 9:283-9.: 283-289.
Eckert RL (1989) Structure, function, and differentiation of the keratinocyte. Physiol Rev 69: 1316-1346.
Eichholtz T, de Bont DB, de WJ, Liskamp RM, and Ploegh HL (1993) A myristoylated pseudosubstrate peptide, a novel protein kinase C inhibitor. J Biol Chem 268: 1982-1986. Erangionic, Neel (1993) Analytical Biochemistry, 210:179.
Freeman (1969) San Francisco, pp. 27-62.
Fuchs E and Byrne C (1994) The epidermis: rising to the surface. Curr Opin Genet Dev 4: 725-736.
Gartsbein M, Alt A, Hashimoto K, Nakajima K, Kuroki T, and Tennenbaum T (2006) The role of protein kinase C delta activation and STAT3 Ser727 phosphorylation in insulin-induced keratinocyte proliferation. J Cell Sci 119: 470-481.
Goldsmith, L. A.
Goodson WH and Hunt TK (1979) Wound healing and the diabetic patient. Surg Gynecol Obstet 149: 600-608.
Green H (1977) Terminal differentiation of cultured human epidermal cells. Cell 11: 405-416. Grunfeld C (1992) Diabetic foot ulcers: etiology, treatment, and prevention. Adv Intern Med 37:103-32: 103-132.
Hennings H, Michael D, Cheng C, Steinert P, Holbrook K, and Yuspa SH (1980) Calcium regulation of growth and differentiation of mouse epidermal cells in culture. Cell 19: 245-254.
Hofmann J (1997) The potential for isoenzyme-selective modulation of protein kinase C. FASEB J 11: 649-669.
Houghten (1985) Proc. Natl. Acad. Sci., USA 82:5131.
House C and Kemp BE (1987) Protein kinase C contains a pseudosubstrate prototope in its regulatory domain. Science 238: 1726-1728.
Jones KT and Sharpe GR (1994) Staurosporine, a non-specific PKC inhibitor, induces keratinocyte differentiation and raises intracellular calcium, but Ro31-8220, a specific inhibitor, does not. J. Cell Physiol 159: 324-330.
Kazanietz MG, Areces LB, Bahador A, Mischak H, Goodnight J, Mushinski JF, and Blumberg PM (1993) Characterization of ligand and substrate specificity for the calcium-dependent and calcium-independent PKC isozymes. Mol Pharmacol 44:298-307.
Keast DH and Orsted H (1998) The basic principles of wound care. Ostomy Wound Manage 44: 24-1.
Kikkawa U, Kishimoto A, and Nishizuka Y (1989) The protein kinase C family: heterogeneity and its implications. Annu Rev Biochem 58: 31-44.
Kirsner RS and Eaglstein WH (1993) The wound healing process. Dermatol Clin 11: 629-640. Knol BW and Wisselink MA (1996) Lick granuloma in dogs; an obsession for dogs, owners and veterinarians. Tijdschr Diergeneeskd 121: 21-23.
Li L, Tennenbaum T, and Yuspa SH (1996) Suspension-induced murine keratinocyte differentiation is mediated by calcium. J Invest Dermatol 106: 254-260.
Li W, Nadelman C, Gratch NS, Chen M, Kasahara N, and Woodley DT (2002) An important role for protein kinase C-delta in human keratinocyte migration on dermal collagen. Exp Cell Res 273: 219-228.
Merrifield (1962) 85 J. Am. Chem. Soc. 2149.
Mousley M (2003) Diabetes and its effect on wound healing and patient care. Nurs Times 99: 70, 73-70,74.
Nash LG, Phillips MN, Nicholson H, Barnett R, and Zhang M (2004) Skin ligaments: regional distribution and variation in morphology. Clin Anat 17: 287-293.
Nishizuka Y (1988) The molecular heterogeneity of PKC and its implications for cellular regulation. Nature 334: 661-665.
Nishizuka Y (1995) Protein kinase C and lipid signaling for sustained cellular responses. FASEB J 9: 484-496.
Ohba M, Ishino K, Kashiwagi M, Kawabe S, Chida K, Huh NH, and Kuroki T (1998) Induction of differentiation in normal human keratinocytes by adenovirus-mediated introduction of the eta and delta isoforms of protein kinase C. Mol Cell Biol 18: 5199-5207.
Querleux B, Cornillon C, Jolivet O, and Bittoun J (2002) Anatomy and physiology of subcutaneous adipose tissue by in vivo magnetic resonance imaging and spectroscopy: relationships with sex and presence of cellulite. Skin Res Technol 8: 118-124.
Saito I, Oya Y, Yamamoto K, Yuasa T, Shimojo H. (1985) Construction of nondefective adenovirus type 5 bearing a 2.8-kilobase hepatitis B virus DNA near the right end of its genome. J Virol. Jun;54(3):711-719.
Sambrook J, Fritsch EF, Maniatis T (2001) Molecular Cloning: A Laboratory Manual (3d ed.). Seo HR, Kwan YW, Cho CK, Bae S, Lee SJ, Soh JW, Chung HY, and Lee YS (2004) PKCalpha induces differentiation through ERK1/2 phosphorylation in mouse keratinocytes. Exp Mol Med 36: 292-299.
Shaw JE and Boulton AJ (1997) The pathogenesis of diabetic foot problems: an overview. Diabetes 46 Suppl 2:S58-61: S58-S61.
Shen S, Alt A, Wertheimer E, Gartsbein M, Kuroki T, Ohba M, Braiman L, Sampson SR, and Tennenbaum T (2001) PKCdelta activation: a divergence point in the signaling of insulin and IGF-1-induced proliferation of skin keratinocytes. Diabetes 50: 255-264.
Silhi N (1998) Diabetes and wound healing. J Wound Care 7: 47-51.
Stone OJ (1986) Hyperinflammatory proliferative (blastomycosis-like) pyodermas: review, mechanisms, and therapy. J Dermatol Surg Oncol 12: 271-273.
Svetek J, Schara M, Pecar S, Hergenhahn M, and Hecker E (1995) Spectroscopic characterization of specific phorbol ester binding to PKC-receptor sites in membranes in situ. Carcinogenesis 16: 2589-2592.
Tennenbaum T, Yuspa SH, Knox B, Sobel ME, Castronovo V, Yamada Y, and De Luca LM (1991) Alterations in attachment to laminin and localization of laminin binding proteins during differentiation of primary mouse keratinocytes in vitro (abstract). J Invest Dermatol 96: Abstract 566.
White SD (1990) Naltrexone for treatment of acral lick dermatitis in dogs. J Am Vet Med Assoc 196: 1073-1076.
Williams RL and Armstrong DG (1998) Wound healing. New modalities for a new millennium. Clin Podiatr Med Surg 15: 117-128.
Wysocki AB (1999) Skin anatomy, physiology, and pathophysiology. Nurs Clin North Am 34: 777-97, v.
Yeruham I, Gur Y, and Harmelin A (1992) Acral lick dermatitis in a dairy cow. Vet Rec 130: 479-480.
Yim VW, Yeung JH, Mak PS, Graham CA, Lai PB, and Rainer TH (2007) Five year analysis of Jockey Club horse-related injuries presenting to a trauma centre in Hong Kong. Injury 38: 98-103.
Yuspa SH, Hawley-Nelson P, Stanley JR, and Hennings H (1980) Epidermal cell culture. Transplant Proc 12: 114-122.

Preferred embodiments of the invention:
1. A composition comprising a delta-PKC activator, an alpha-PKC inhibitor, and a pharmaceutically acceptable carrier that is free of Ca²⁺ and Mg²⁺ cations.
2. The composition of item 1 wherein the delta-PKC activator is at least one selected from the group consisting of an insulin and an insulin analog.
3. The composition of item 2 wherein the insulin analog is at least one selected from the group consisting of insulin lispro, insulin aspart, insulin glargine, visfatin, and L-α-phosphatidylinositol-3,4,5-triphosphate, dipalmitoyl-, heptaammonium salt
4. The composition of item 2 wherein the insulin is at least one selected from the group consisting of human insulin, bovine insulin, and porcine insulin.
5. The composition of item 4 wherein the insulin is recombinantly expressed.
6. The composition of item 2 wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen.
7. The composition of item 2 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO:12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
8. The composition of item 2 wherein the alpha-PKC inhibitor is a peptide consisting of the amino acid sequence shown in SEQ ID NO: 25 which has a myristoylated amino acid residue at its amino terminus and is amidated at its carboxy terminus.
9. The composition of item 2 wherein the alpha-PKC inhibitor is a peptide consisting of the amino acid sequence shown in SEQ ID NO: 1 which has a myristoylated amino acid residue at its amino terminus.
10. The composition of item 1, 2, 3, 4, 5, 6, 7, 8, or 9 wherein the pharmaceutically acceptable carrier that is free of Ca²⁺ and Mg²⁺ cations is an aqueous carrier comprising 0.2 g/L KCl, 0.2 g/L anhydrous KH₂PO₄, 8 g/L NaCl, and 1.15 g/L anhydrous Na₂HPO₄.
11. A composition comprising an insulin, a peptide consisting of the amino acid sequence shown in SEQ ID NO: 1 which has a myristoylated amino acid residue at its amino terminus, and an aqueous pharmaceutically acceptable carrier comprising 0.2 g/L KCl, 0.2 g/L anhydrous KH₂PO₄, 8 g/L, NaCl, and 1.15 g/L anhydrous Na₂HPO₄ that is free of Ca²⁺ and Mg²⁺ cations.
12. The composition of item 11 comprising about 0.0001 units/L to about 0.1 units/L of insulin and about 1 µM to about 100 µM of the peptide.
13. The composition of item 12 comprising 0.0001 units/L of insulin and 1 µM of the peptide.
14. A composition comprising a delta-PKC activator, an alpha-PKC inhibitor, a pharmaceutically acceptable carrier that is free of Ca²⁺ and Mg²⁺ cations, and a drug eluting scaffold.
15. The composition of item 14 wherein the drug eluting scaffold comprises a porous solid.
16. The composition of item 15 wherein the delta-PKC activator is at least one selected from the group consisting of an insulin and an insulin analog.
17. The composition of item 16 wherein the insulin analog is at least one selected from the group consisting of insulin lispro, insulin aspart, insulin glargine, visfatin, and L-α-phosphatidylinositol-3,4,5-trisphophate, dipalmitoyl-, heptaammonium salt.
18. The composition of item 16 wherein the insulin is at least one selected from the group consisting of human insulin, bovine insulin, and porcine insulin.
19. The composition of item 18 wherein the insulin is recombinantly expressed.
20. The composition of item 16 wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen.
21. The composition of item 16 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
22. The composition of item 16 wherein the alpha-PKC inhibitor is a peptide consisting of the amino acid sequence shown in SEQ ID NO: 25 which has a myristoylated amino acid residue at its amino terminus and is amidated at its carboxy terminus.
23. The composition of item 16 wherein the alpha-PKC inhibitor is a peptide consisting of the amino acid sequence shown in SEQ ID NO: 1 which has a myristoylatod amino acid residue at its amino terminus.
24. The composition of item 23 comprising about 0.0001 units/L to about 0.1 units/L of insulin and about 1 µM to about 100 µM of the peptide.
25. The composition of item 23 comprising 0.0001 units/L of insulin and 1 µM of the peptide.
26. The composition of item 14, 15, 16, 17, 18, 19, 20, 21, 22, 23, 24, or 25 wherein the drug eluting scaffold is a sponge.
27. The composition of item 26 comprising an aqueous pharmaceutically acceptable carrier comprising 0.2 g/L KCI, 0.2 g/L anhydrous KH₂PO₄, 8 g/L NaCl, and 1.15 g/L anhydrous Na₂HPO₄ that is free of Ca²⁺ and Mg²⁺ cations.
28. A pharmaceutical composition produced by a process comprising the steps of:
   a) providing a delta-PKC activator, an alpha-PKC inhibitor, and a pharmaceutically acceptable carrier that is free of Ca²⁺ and Mg²⁺ cations; and
   b) combining the delta-PKC activator, alpha-PKC inhibitor, and the pharmaceutically acceptable carrier that is free of Ca²⁺ and Mg²⁺ cations;
      whereby the pharmaceutical composition is produced.
29. The pharmaceutical composition of item 28 wherein the delta-PKC activator is at least one selected from the group consisting of an insulin and an insulin analog.
30. The pharmaceutical composition of item 29 wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt.
31. The pharmaceutical composition of item 39 wherein the insulin is at least one selected from the group consisting of human insulin, bovine insulin, and porcine insulin.
32. The pharmaceutical composition ofitem 31 wherein the insulin is recombinantly expressed.
33. The pharmaceutical composition of item 29 wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen.
34. The pharmaceutical composition of item 29 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
35. The pharmaceutical composition of item 29 wherein the alpha-PKC inhibitor is a peptide consisting of the amino acid sequence shown in SEQ ID NO: 25 which has a myristoylated amino acid residue at its amino terminus and is amidated at its carboxy terminus.
36. The pharmaceutical composition of item 29 wherein the alpha-PKC inhibitor is a peptide consisting of the amino acid sequence shown in SEQ ID NO: 1 which has a myristoylated amino acid residue at its amino terminus.
37. The pharmaceutical composition of item 36 comprising about 0.0001 units/L to about 0.1 units/L of insulin and about 1 µM to about 100 µM of the peptide.
38. The pharmaceutical composition of item 36 comprising 0.0001 units/L of insulin and 1 µM of the peptide.
39. The pharmaceutical composition of item 28, 29, 30, 31, 32, 33, 34, 35, 36, 37, or 38 wherein the pharmaceutically acceptable carrier that is free of Ca²⁺ and Mg²⁺ cations is an aqueous carrier comprising 0.2 g/L KCI, 0.2 g/L anhydrous KH₂PO₄, 8 g/L NaCl, and 1.15 g/L anhydrous Na₂HPO₄.
40. A method for increasing the closure of a skin wound on an animal comprising the steps of:
   a) providing a pharmaceutical composition comprising a delta-PKC activator, an alpha-PKC inhibitor, and a pharmaceutically acceptable carrier that is free of Ca²⁺ and Mg²⁺ cations; and
   b) administering to a skin wound on an animal an effective amount of the pharmaceutical composition;
      whereby closure of the skin wound is increased.
41. The method of item 40 wherein the delta-PKC activator is at least one selected from the group consisting of an insulin and an insulin analog.
42. The method of item 41 wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt.
43. The method of item 41 wherein the insulin is at least one selected from the group consisting of human insulin, bovine insulin, and porcine insulin.
44. The method of item 43 wherein the insulin is recombinantly expressed.
45. The method of item 41 wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen.
46. The method of item 41 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
47. The method of item 41 wherein the alpha-PKC inhibitor is a peptide consisting of the amino acid sequence shown in SEQ ID NO: 25 which has a myristoylated amino acid residue at its amino terminus and is amidated at its carboxy terminus.
48. The method of item 41 wherein the alpha-PKC inhibitor is a peptide consisting of the amino acid sequence shown in SEQ ID NO: 1 which has a myristoylated amino acid residue at its amino terminus.
49. The method of item 48 wherein the pharmaceutical composition comprises about 0.0001 units/L to about 0.1 units/L of insulin and about 1 µM to about 100 µM of the peptide.
50. The method of item 48 wherein the pharmaceutical composition comprises 0.0001 units/L of insulin and 1 µM of the peptide.
51. The method of items 40, 41, 42, 43, 44, 45, 46, 47, 48, 49, or 50 wherein the pharmaceutically acceptable carrier that is free of Ca²⁺ and Mg²⁺ cations is an aqueous carrier comprising 0.2 g/L KCI, 0.2 g/L anhydrous KH₂PO₄, 8 g/L NaCl, and 1.15 g/L anhydrous Na₂HPO₄.
52. A method for decreasing inflammation at the site of a skin wound on an animal comprising the steps of:
   a) providing a pharmaceutical composition comprising a delta-PKC activator, an alpha-PKC inhibitor, and a pharmaceutically acceptable carrier that is free of Ca²⁺ and Mg²⁺ cations; and
   b) administering to a skin wound on an animal an effective amount of the pharmaceutical composition;
      whereby inflammation at the site of the skin wound is decreased.
53. The method of item 52 wherein the delta-PKC activator is at least one selected from the group consisting of an insulin and an insulin analog.
54. The method of item 53 wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt.
55. The method of item 53 wherein the insulin is at least one selected from the group consisting of human insulin, bovine insulin, and porcine insulin.
56. The method of item 55 wherein the insulin is recombinantly expressed.
57. The method of item 53 wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen.
58. The method of item 53 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
59. The method of item 52 wherein the alpha-PKC inhibitor is a peptide consisting of the amino acid sequence shown in SEQ ID NO: 25 which has a myristoylated amino acid residue at its amino terminus and is amidated at its carboxy terminus.
60. The method of item 52 wherein the alpha-PKC inhibitor is a peptide consisting of the amino acid sequence shown in SEQ ID NO: 1 which has a myristoylated amino acid residue at its amino terminus.
61. The method of item 60 wherein the pharmaceutical composition comprises about 0.0001 units/L to about 0.1 units/L of insulin and about 1 µM to about 100 µM of the peptide.
62. The method of item 60 wherein the pharmaceutical composition comprises 0.0001 units/L of insulin and 1 µM of the peptide.
63. The method of items 52, 53, 54,55, 56, 57, 58, 59, 60, 61, or 62 wherein the pharmaceutically acceptable carrier that is free of Ca²⁺ and Mg²⁺ cations is an aqueous carrier comprising 0.2 g/L KCl, 0.2 g/L anhydrous KH₂PO₄, 8 g/L NaCl, and 1.15 g/L anhydrous Na₂HPO₄.
64. A composition comprising an insulin or an insulin analog and a pharmaceutically acceptable carrier that is free of Ca²⁺ and Mg²⁺ cations.
65. The composition of item 64 wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt.
66. The composition of item 64 wherein the insulin is at least one selected from the group consisting of human insulin, bovine insulin, and porcine insulin.
67. The composition of item 66 wherein the insulin is recombinantly expressed.
68. The composition of item 64 comprising about 0.0001 units/L to about 0.1 units/L of an insulin or an insulin analog.
69. The composition of item 64 comprising 0.0001 units/L of an insulin or an insulin analog.
70. The composition of items 64, 65, 66, 67, 68, or 69 wherein the pharmaceutically acceptable carrier that is free of Ca²⁺ and Mg²⁺ cations is an aqueous carrier comprising 0.2 g/L KCl, 0.2 g/L anhydrous KH₂PO₄, 8 g/L NaCl, and 1.15 g/L anhydrous Na₂HPO₄.
71. A composition comprising 0.0001 units/L to about 0.1 units/L of an insulin and a pharmaceutically acceptable carrier that is free of Ca²⁺ and Mg²⁺ cations.
72. The composition of item 71 wherein the insulin is at least one selected from the group consisting of human insulin, bovine insulin, and porcine insulin.
73. The composition of item 72 wherein the insulin is recombinantly expressed.
74. The composition of items 71, 72, or 73 wherein the pharmaceutically acceptable carrier that is free of Ca²⁺ and Mg²⁺ cations is an aqueous carrier comprising 0.2 g/L KCl, 0.2 g/L anhydrous KH₂PO₄, 8 g/L NaCl, and 1.15 g/L anhydrous Na₂HPO₄.
75. A method for increasing the closure of a wound on an animal comprising the steps of:
   a) providing a pharmaceutical composition comprising a delta-PKC activator, an alpha-PKC inhibitor, and a pharmaceutically acceptable carrier that is free of Ca²⁺ and Mg²⁺ cations; and
   b) administering to a wound on an animal an effective amount of the pharmaceutical composition, wherein the wound is at least one selected from the group consisting of diabetic ulcer wounds, acral lick wounds, proud flesh wounds, surgical wounds, chronic solar abscess wounds, and osteomyelitis wounds;
      whereby closure of the wound is increased.
76. The method of item 75 wherein the delta-PKC activator is at least one selected from the group consisting of an insulin and an insulin analog.
77. The method of item 76 wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt.
78. The method of item 76 wherein the insulin is at least one selected from the group consisting of human insulin, bovine insulin, and porcine insulin.
79. The method of item 78 wherein the insulin is recombinantly expressed.
80. The method of item 76 wherein the alpha-PKC inhibitor is at least one selected from the group consisting of (S)-2,6-Diamino-N-[(1-(1-oxotridecyl)-2 piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole ; 2-[1-(3-Dimethylaminopropyl)-1H-indol-3-yl]-3-(1H-indol-3-yl) maleimide; and aprinocarsen.
81. The method of item 76 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
82. The method of item 76 wherein the alpha-PKC inhibitor is a peptide consisting of the amino acid sequence shown in SEQ ID NO: 25 which has a myristoylated amino acid residue at its amino terminus and is amidated at its carboxy terminus.
83. The method of item 76 wherein the alpha-PKC inhibitor is a peptide consisting of the amino acid sequence shown in SEQ ID NO: 1 which has a myristoylated amino acid residue at its amino terminus.
84. The method of item 83 wherein the pharmaceutical composition comprises about 0.0001 units/L to about 0.1 units/L of insulin and about 1 µM to about 100 µM of the peptide.
85. The method of item 83 wherein the pharmaceutical composition comprises 0.0001 units/L of insulin and 1 µM of the peptide.
86. The method of items 75, 76, 77, 78, 79, 80, 81, 82, 83, 84, or 85 wherein the pharmaceutically acceptable carrier that is free of Ca²⁺ and Mg²⁺ cations is an aqueous carrier comprising 0.2 g/L KCI, 0.2 g/L anhydrous KH₂PO₄, 8 g/L NaCl, and 1.15 g/L anhydrous Na₂HPO₄.
87. A composition comprising a delta-PKC activator, an alpha-PKC inhibitor, and a pharmaceutically acceptable carrier that contains K⁺ cations and is free of Ca²⁺ and Mg²⁺ cations.
88. The composition of item 87 wherein the delta-PKC activator is at least one selected from the group consisting of an insulin and an insulin analog.
89. The composition of item 88 wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt.
90. The composition of item 88 wherein the insulin is at least one selected from the group consisting of human insulin, bovine insulin, and porcine insulin.
91. The composition of item 90 wherein the insulin is recombinantly expressed.
92. The composition of item 88 wherein the alpha-PKC inhibitor is at least one selected from the group consisting of (S)-2,6-Diamino-N-[(1-(1-oxotridecyl-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen.
93. The composition of item 88 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
94. The composition of item 88 wherein the alpha-PKC inhibitor is a peptide consisting of the amino acid sequence shown in SEQ ID NO: 25 which has a myristoylated amino acid residue at its amino terminus and is amidated at its carboxy terminus.
95. The composition of item 88 wherein the alpha-PKC inhibitor is a peptide consisting of the amino acid sequence shown in SEQ ID NO: 1 which has a myristoylated amino acid residue at its amino terminus.
96. A composition comprising a delta-PKC activator and a pharmaceutically acceptable carrier that contains K⁺ cations and is free of Ca²⁺ and Mg²⁺ cations.
97. The composition of item 96 wherein the delta-PKC activator is at least one selected from the group consisting of an insulin and an insulin analog.
98. The composition of item 97 wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt.
99. The composition of item 97 wherein the insulin is at least one selected from the group consisting of human insulin, bovine insulin, and porcine insulin.
100. The composition of item 99 wherein the insulin is recombinantly expressed.
101. A composition comprising an alpha-PKC inhibitor, and a pharmaceutically acceptable carrier that is free of Ca²⁺ and Mg²⁺ cations.
102. The composition of item 101 wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen.
103. The composition of item 101 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ m NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
104. The composition of item 101 wherein the alpha-PKC inhibitor is a peptide consisting of the amino acid sequence shown in SEQ ID NO: 25 which has a myristoylated amino acid residue at its amino terminus and is amidated at its carboxy terminus.
105. The composition of item 101 wherein the alpha-PKC inhibitor is a peptide consisting of the amino acid sequence shown in SEQ ID NO: 1 which has a myristoylated amino acid residue at its amino terminus.
106. The composition of item 105 wherein the pharmaceutical composition comprises about 1 µM to about 100 µM of the peptide.
107. The composition of item 106 wherein the pharmaceutical composition comprises 1 µM of the peptide.
108. The composition of items 101, 102, 103, 104, 105, 105, 106, or 107 wherein the pharmaceutically acceptable carrier that is free of Ca²⁺ and Mg²⁺ cations is an aqueous carrier comprising 0.2 g/L KCl, 0.2 g/L anhydrous KH₂PO₄, 8 g/L NaCl, and 1.15 g/L anhydrous Na₂HPO₄.
109. A method for decreasing inflammation at the site of a skin wound on an animal comprising the steps of:
   a) providing a pharmaceutical composition comprising an alpha-PKC inhibitor and a pharmaceutically acceptable carrier that is free of Ca²⁺ and Mg²⁺ cations; and
   b) administering to a skin wound on an animal an effective amount of the pharmaceutical composition;
      whereby inflammation at the site of the skin wound is decreased.
110. The method of item 109 wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoemyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen.
111. The method of item 109 wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55.
112. The method of item 109 wherein the alpha-PKC inhibitor is a peptide consisting of the amino acid sequence shown in SEQ ID NO: 25 which has a myristoylated amino acid residue at its amino terminus and is amidated at its carboxy terminus.
113. The method of item 109 wherein the alpha-PKC inhibitor is a peptide consisting of the amino acid sequence shown in SEQ ID NO: 1 which has a myristoylated amino acid residue at its amino terminus.
114. The method of item 109 wherein the pharmaceutical composition comprises about 1 µM to about 100 µM of the peptide.
115. The method of item 114 wherein the pharmaceutical composition comprises 1 µM of the peptide.
116. The method of items 107, 108, 109, 110, 112, 113, 114, or 115 wherein the pharmaceutically acceptable carrier that is free of Ca²⁺ and Mg²⁺ cations is an aqueous carrier comprising 0.2 g/L KCI, 0.2 g/L anhydrous KH₂PO₄, 8 g/L NaCl, and 1.15 g/L anhydrous Na₂HPO₄.

## Claims

1. A composition comprising a delta-PKC activator, an alpha-PKC inhibitor, and a pharmaceutically acceptable carrier that is free of Ca²⁺ and Mg²⁺ cations,
wherein the delta-PKC activator is at least one selected from the group consisting of an insulin and an insulin analog, preferably
wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt, preferably
2 wherein the insulin is at least one selected from the group consisting of human insulin, bovine insulin, and porcine insulin, preferably
wherein the insulin is recombinantly expressed , and
wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimemylaminopropyl)-1H-indol-3-yl]-3-(1H-indol-3-yl) maleimide; and aprinocarsen, or
wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55, preferably
wherein the alpha-PKC inhibitor is a peptide consisting of the amino acid sequence shown in SEQ ID NO: 25 which has a myristoylated amino acid residue at its amino terminus and is amidated at its carboxy terminus, or
wherein the alpha-PKC inhibitor is a peptide consisting of the amino acid sequence shown in SEQ ID NO: 1 which has a myristoylated amino acid residue at its amino terminus, preferably
wherein the pharmaceutically acceptable carrier that is free of Ca²⁺ and Mg²⁺ cations is an aqueous carrier comprising 0.2 g/L KCl, 0.2 g/L anhydrous KH₂PO₄, 8 g/L NaCl, and 1.15 g/L anhydrous Na₂HPO₄.

2. A composition comprising an insulin, a peptide consisting of the amino acid sequence shown in SEQ ID NO: 1 which has a myristoylated amino acid residue at its amino terminus, and an aqueous pharmaceutically acceptable carrier comprising 0.2 g/L KCl, 0.2 g/L anhydrous KH₂PO₄, 8 g/L NaCl, and 1.15 g/L anhydrous Na₂HPO₄ that is free of Ca²⁺ and Mg²⁺ cations , and
comprising about 0.0001 units/L to about 0.1 units/L of insulin and about 1 µM to about 100 µM of the peptide, preferably
comprising 0.0001 units/L of insulin and 1 µM of the peptide.

3. A composition comprising a delta-PKC activator, an alpha-PKC inhibitor, a pharmaceutically acceptable carrier that is free of Ca²⁺ and Mg²⁺ cations, and a drug eluting scaffold, Preferably
wherein the drug eluting scaffold comprises a porous solid, and wherein the delta-PKC activator is at least one selected from the group consisting of an insulin and an insulin analog, preferably
wherein the insulin analog is at least one selected from the group consisting of insulin lispro, insulin aspart, insulin glargine, visfatin, and L-α-phosphatidylinositol-3,4,5-trisphosphate, dipalmitoyl-, heptaammonium salt, preferably
wherein the insulin is at least one selected from the group consisting of human insulin, bovine insulin, and porcine insulin, preferably
wherein the insulin is recombinantly expressed, and
wherein the alpha-PKC inhibitor is at least one selected from the group consisting of (S)-2,6-Diamino-N-[(1-(1-oxotridecyl)-2-piperidinyl)methyl]hexanamide dihydrochloride hydrate; 4'-N-Benzoyl Staurosporine; Bisindolylmaleimide IX, Methanesulfonate salt; 12-(2-cyanoethyl)-6,7,12,13-tetrahydro-13-methyl-5-oxo-5H-indolo[2,3-a]pyrrollo[3,4-c]carbazole; 2-[1-(3-Dimethylaminopropyl)-1H-indol-3-yl]-3-(1H-indol-3-yl) maleimide; and aprinocarsen, or
wherein the alpha-PKC inhibitor is at least one selected from the group consisting of a peptide having the amino acid sequence shown in SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 55, preferably
wherein the alpha-PKC inhibitor is a peptide consisting of the amino acid sequence shown in SEQ ID NO: 25 which has a myristoylated amino acid residue at its amino terminus and is amidated at its carboxy terminus, or
wherein the alpha-PKC inhibitor is a peptide consisting of the amino acid sequence shown in SEQ ID NO: I which has a myristoylated amino acid residue at its amino terminus, and
comprising about 0.0001 units/L to about 0.1 units/L of insulin and about 1 µM to about 100 µM of the peptide, preferably
comprising 0.0001 units/L of insulin and 1 µM of the peptide, preferably
wherein the drug eluting scaffold is a sponge, preferably
comprising an aqueous pharmaceutically acceptable carrier comprising 0.2 g/L KCl, 0.2 g/L anhydrous KH₂PO₄, 8 g/L NaCl, and 1.15 g/L anhydrous Na₂HPO₄ that is free of Ca²⁺ and Mg²⁺ cations.

4. A pharmaceutical composition produced by a process comprising the steps of:
a) providing a delta-PKC activator, and an alpha-PKC inhibitor according to any of claims 1-3, and a pharmaceutically acceptable carrier that is free of Ca²⁺ and Mg²⁺ cations; and
b) combining the delta-PKC activator, alpha-PKC inhibitor, and the pharmaceutically acceptable carrier that is free of Ca²⁺ and Mg²⁺ cations;
whereby the pharmaceutical composition is produced.

5. A composition of claims 1, 2 or 3 for use in increasing the closure of a skin wound on an animal.

6. A composition of claims 1, 2 or 3 for use in decreasing inflammation at the site of a skin wound on an animal.

7. A composition of claims 1, 2 or 3 for use in increasing the closure of a wound on an animal.

8. A composition of claims 1, 2 or 3 for use in decreasing inflammation at the site of a skin wound on an animal.


SEQUENCE LISTING

<110> HEALOR LTD.

<120> PHARMACEUTICAL COMPOSITION

<130>

<140>
<141>

<150> 60/962,706
<151> 2007-07-30

<160> 64

<170> PatentIn version 3.3

<210> 1
<211> 9
<212> PRT
<213> Artificial Sequence

<220>
<223> Description of Artificial Sequence: Synthetic
      peptide

<220>
<223> N-term myristoylated

<400> 1
Phe Ala Arg Lys Gly Ala Leu Arg Gln
1               5


<210> 2
<211> 13
<212> PRT
<213> Artificial Sequence

<220>
<223> Description of Artificial Sequence: Synthetic
      peptide

<400> 2
Arg Phe Ala Arg Lys Gly Ala Leu Arg Gln Lys Asn Val
1               5                   10


<210> 3
<211> 18
<212> PRT
<213> Artificial Sequence

<220>
<223> Description of Artificial Sequence: Synthetic
      peptide

<400> 3
Arg Phe Ala Arg Lys Gly Ala Leu Arg Gln Lys Asn Val His Glu Val
1               5                   10                  15

Lys Asn

<210> 4
<211> 22
<212> PRT
<213> Artificial Sequence

<220>
<223> Description of Artificial Sequence: Synthetic
      peptide

<400> 4
Arg Phe Ala Arg Lys Gly Ala Leu Arg Gln Lys Asn Val His Glu Val
1               5                   10                  15

Lys Asn Leu Lys Gly Ala
            20

<210> 5
<211> 13
<212> PRT
<213> Artificial Sequence

<220>
<223> Description of Artificial Sequence: Synthetic
      peptide

<400> 5
Arg Phe Ala Arg Lys Gly Ala Leu Arg Gln Leu Ala Val
1               5                   10

<210> 6
<211> 13
<212> PRT
<213> Artificial Sequence

<220>
<223> Description of Artificial Sequence: Synthetic
      peptide

<400> 6
Arg Phe Ala Arg Lys Gly Ala Leu Ala Gln Lys Asn Val
1               5                   10

<210> 7
<211> 9
<212> PRT
<213> Artificial Sequence

<220>
<223> Description of Artificial Sequence: Synthetic
      peptide

<400> 7

Arg Phe Ala Arg Lys Gly Ala Leu Arg
1               5

<210> 8
<211> 13
<212> PRT
<213> Artificial Sequence

<220>
<223> Description of Artificial Sequence: Synthetic
      peptide

<220>
<221> MOD_RES
<222> (3)..(3)
<223> Any amino acid

<400> 8
Tyr Tyr Xaa Lys Arg Lys Met Ala Phe Phe Glu Phe Phe
1               5                   10

<210> 9
<211> 13
<212> PRT
<213> Artificial Sequence

<220>
<223> Description of Artificial Sequence: Synthetic
      peptide

<400> 9
Phe Lys Leu Lys Arg Lys Gly Ala Phe Lys Lys Phe Ala
1               5                   10

<210> 10
<211> 13
<212> PRT
<213> Artificial Sequence

<220>
<223> Description of Artificial Sequence: Synthetic
      peptide

<400> 10
Ala Arg Arg Lys Arg Lys Gly Ala Phe Phe Tyr Gly Gly
1               5                   10

<210> 11
<211> 13
<212> PRT
<213> Artificial Sequence

<220>
<223> Description of Artificial Sequence: Synthetic
      peptide

<400> 11

Arg Arg Arg Arg Arg Lys Gly Ala Phe Arg Arg Lys Ala
1               5                   10

<210> 12
<211> 14
<212> PRT
<213> Artificial Sequence

<220>
<223> Description of Artificial Sequence: Synthetic
      peptide

<400> 12
Arg Phe Ala Arg Lys Gly Ala Leu Arg Gln Lys Asn Val Tyr
1               5                   10

<210> 13
<211> 12
<212> PRT
<213> Artificial Sequence

<220>
<223> Description of Artificial Sequence: Synthetic
      peptide

<400> 13
Asp Ala Arg Lys Gly Ala Leu Arg Gln Asn Lys Val
1               5                   10

<210> 14
<211> 16
<212> PRT
<213> Artificial Sequence

<220>
<223> Description of Artificial Sequence: Synthetic
      peptide

<400> 14
Glu Arg Met Arg Pro Arg Lys Arg Gln Gly Ala Val Arg Arg Arg Val
1               5                   10                  15

<210> 15
<211> 17
<212> PRT
<213> Artificial Sequence

<220>
<223> Description of Artificial Sequence: Synthetic
      peptide

<400> 15
Gly Pro Arg Pro Leu Phe Cys Arg Lys Gly Ala Leu Arg Gln Lys Val
1               5                   10                  15

Val

<210> 16
<211> 11
<212> PRT
<213> Artificial Sequence

<220>
<223> Description of Artificial Sequence: Synthetic
      peptide

<400> 16
Gln Lys Arg Pro Ala Gln Arg Ser Lys Tyr Leu
1               5                   10


<210> 17
<211> 11
<212> PRT
<213> Artificial Sequence

<220>
<223> Description of Artificial Sequence: Synthetic
      peptide

<400> 17
Gln Lys Arg Pro Ser Gln Arg Ala Lys Tyr Leu
1               5                   10


<210> 18
<211> 12
<212> PRT
<213> Artificial Sequence

<220>
<223> Description of Artificial Sequence: Synthetic
      peptide

<400> 18
Gly Gly Pro Leu Arg Arg Thr Leu Ala Val Arg Arg
1               5                   10


<210> 19
<211> 12
<212> PRT
<213> Artificial Sequence

<220>
<223> Description of Artificial Sequence: Synthetic
      peptide

<400> 19
Gly Gly Pro Leu Ser Arg Arg Leu Ala Val Arg Arg
1               5                   10


<210> 20
<211> 12
<212> PRT

<213> Artificial Sequence

<220>
<223> Description of Artificial Sequence: Synthetic
      peptide

<400> 20
Gly Gly Pro Leu Ser Arg Thr Leu Ala Val Arg Arg
1               5                   10


<210> 21
<211> 12
<212> PRT
<213> Artificial Sequence

<220>
<223> Description of Artificial Sequence: Synthetic
      peptide

<400> 21
Gly Gly Pro Leu Ser Arg Arg Leu Ala Val Ala Arg
1               5                   10


<210> 22
<211> 12
<212> PRT
<213> Artificial Sequence

<220>
<223> Description of Artificial Sequence: Synthetic
      peptide

<400> 22
Gly Gly Pro Leu Arg Arg Thr Leu Ala Val Ala Arg
1               5                   10


<210> 23
<211> 8
<212> PRT
<213> Artificial Sequence

<220>
<223> Description of Artificial Sequence: Synthetic
      peptide

<400> 23
Val Arg Lys Ala Leu Arg Arg Leu
1               5


<210> 24
<211> 12
<212> PRT
<213> Artificial Sequence

<220>
<223> Description of Artificial Sequence: Synthetic
      peptide

<400> 24
Gly Gly Arg Leu Ser Arg Thr Leu Ala Val Ala Arg
1               5                   10

<210> 25
<211> 17
<212> PRT
<213> Artificial Sequence

<220>
<223> Description of Artificial Sequence: Synthetic
      peptide

<220>
<223> N-term myristoylated

<220>
<223> C-term amidated

<400> 25

Thr Arg Lys Arg Gln Pro Ala Met Arg Arg Arg Val His Gln Ile Asn
1               5                   10                  15

Gly

<210> 26
<211> 12
<212> PRT
<213> Artificial Sequence

<220>
<223> Description of Artificial Sequence: Synthetic
      peptide

<400> 26
Arg Lys Arg Gln Arg Ala Met Arg Arg Arg Val His
1               5                   10

<210> 27
<211> 16
<212> PRT
<213> Artificial Sequence

<220>
<223> Description of Artificial Sequence: Synthetic
      peptide

<400> 27
Glu Arg Met Arg Pro Arg Lys Arg Gln Gly Ala Val Arg Arg Arg Val
1               5                   10                  15

<210> 28
<211> 13
<212> PRT

<213> Artificial Sequence

<220>
<223> Description of Artificial Sequence: Synthetic
      peptide

<400> 28
Phe Lys Leu Lys Arg Lys Gly Ala Phe Lys Lys Phe Ala
1               5                   10


<210> 29
<211> 13
<212> PRT
<213> Artificial Sequence

<220>
<223> Description of Artificial Sequence: Synthetic
      peptide

<220>
<221> MOD_RES
<222> (3)..(3)
<223> Any amino acid

<400> 29
Tyr Tyr Xaa Lys Arg Lys Met Ala Phe Phe Glu Phe Phe
1               5                   10


<210> 30
<211> 13
<212> PRT
<213> Artificial Sequence

<220>
<223> Description of Artificial Sequence: Synthetic
      peptide

<400> 30
Ala Arg Arg Lys Arg Lys Gly Ala Phe Phe Tyr Gly Gly
1               5                   10


<210> 31
<211> 13
<212> PRT
<213> Artificial Sequence

<220>
<223> Description of Artificial Sequence: Synthetic
      peptide

<400> 31
Arg Arg Arg Arg Arg Lys Gly Ala Phe Arg Arg Lys Ala
1               5                   10


<210> 32
<211> 20
<212> PRT

<213> Artificial Sequence

<220>
<223> Description of Artificial Sequence: Synthetic
      peptide

<400> 32
Ala Ala Ala Lys Ile Gln Ala Ala Trp Arg Gly His Met Ala Arg Lys
1               5                   10                  15

Lys Ile Lys Ser
            20

<210> 33
<211> 19
<212> PRT
<213> Artificial Sequence

<220>
<223> Description of Artificial Sequence: Synthetic
      peptide

<400> 33
Ala Ala Ala Lys Ile Gln Ala Ala Phe Arg Gly His Met Ala Arg Lys
1               5                   10                  15

Lys Ile Lys

<210> 34
<211> 16
<212> PRT
<213> Artificial Sequence

<220>
<223> Description of Artificial Sequence: Synthetic
      peptide

<400> 34
Glu Arg Met Arg Pro Arg Lys Arg Gln Gly Ala Val Arg Arg Arg Val
1               5                   10                  15

<210> 35
<211> 8
<212> PRT
<213> Artificial Sequence

<220>
<223> Description of Artificial Sequence: Synthetic
      peptide

<400> 35
Val Arg Lys Ala Leu Arg Arg Leu
1               5

<210> 36

<211> 22
<212> PRT
<213> Artificial Sequence

<220>
<223> Description of Artificial Sequence: Synthetic
      peptide

<400> 36
Lys Lys Lys Lys Lys Arg Phe Ser Phe Lys Lys Ala Phe Lys Leu Ser
1               5                   10                  15

Gly Phe Ser Phe Lys Lys
            20

<210> 37
<211> 17
<212> PRT
<213> Artificial Sequence

<220>
<223> Description of Artificial Sequence: Synthetic
      peptide

<400> 37
Gly Pro Arg Pro Leu Phe Cys Arg Lys Gly Ala Leu Arg Gln Lys Val
1               5                   10                  15

Val

<210> 38
<211> 17
<212> PRT
<213> Artificial Sequence

<220>
<223> Description of Artificial Sequence: Synthetic
      peptide

<400> 38
Glu Ser Thr Val Arg Phe Ala Arg Lys Gly Ala Leu Arg Gln Lys Asn
1               5                   10                  15

Val

<210> 39
<211> 16
<212> PRT
<213> Artificial Sequence

<220>
<223> Description of Artificial Sequence: Synthetic
      peptide

<400> 39

Glu Arg Met Arg Pro Arg Lys Arg Gln Gly Ala Val Arg Arg Arg Val
1               5                   10                  15

<210> 40
<211> 13
<212> PRT
<213> Artificial Sequence

<220>
<223> Description of Artificial Sequence: Synthetic
      peptide

<400> 40
Arg Phe Ala Arg Leu Gly Ala Leu Arg Gln Lys Asn Val
1               5                   10

<210> 41
<211> 13
<212> PRT
<213> Artificial Sequence

<220>
<223> Description of Artificial Sequence: Synthetic
      peptide

<220>
<221> MOD_RES
<222> (3)..(3)
<223> Any amino acid

<400> 41
Tyr Tyr Xaa Lys Arg Lys Met Ala Phe Phe Glu Phe Phe
1               5                   10

<210> 42
<211> 13
<212> PRT
<213> Artificial Sequence

<220>
<223> Description of Artificial Sequence: Synthetic
      peptide

<400> 42
Arg Arg Phe Lys Arg Gln Gly Ala Phe Phe Tyr Phe Phe
1               5                   10

<210> 43
<211> 13
<212> PRT
<213> Artificial Sequence

<220>
<223> Description of Artificial Sequence: Synthetic
      peptide

<400> 43

Phe Lys Leu Lys Arg Lys Gly Ala Phe Lys Lys Phe Ala
1               5                   10

<210> 44
<211> 13
<212> PRT
<213> Artificial Sequence

<220>
<223> Description of Artificial Sequence: Synthetic
      peptide

<400> 44
Ala Arg Arg Lys Arg Lys Gly Ser Phe Phe Tyr Gly Gly
1               5                   10

<210> 45
<211> 13
<212> PRT
<213> Artificial Sequence

<220>
<223> Description of Artificial Sequence: Synthetic
      peptide

<400> 45
Phe Lys Leu Lys Arg Lys Gly Ser Phe Lys Lys Phe Ala
1               5                   10

<210> 46
<211> 13
<212> PRT
<213> Artificial Sequence

<220>
<223> Description of Artificial Sequence: Synthetic
      peptide

<400> 46
Arg Arg Phe Lys Arg Gln Gly Ser Phe Phe Tyr Phe Phe
1               5                   10

<210> 47
<211> 13
<212> PRT
<213> Artificial Sequence

<220>
<223> Description of Artificial Sequence: Synthetic
      peptide

<220>
<221> MOD_RES
<222> (3)..(3)
<223> Any amino acid

<400> 47

Tyr Tyr Xaa Lys Arg Lys Met Ser Phe Phe Glu Phe Phe
1               5                   10

<210> 48
<211> 13
<212> PRT
<213> Artificial Sequence

<220>
<223> Description of Artificial Sequence: Synthetic
      peptide

<400> 48
Arg Arg Arg Arg Arg Lys Gly Ser Phe Arg Arg Lys Ala
1               5                   10

<210> 49
<211> 16
<212> PRT
<213> Artificial Sequence

<220>
<223> Description of Artificial Sequence: Synthetic
      peptide

<400> 49
Glu Arg Met Arg Pro Arg Lys Arg Gln Gly Ser Val Arg Arg Arg Val
1               5                   10                  15

<210> 50
<211> 12
<212> PRT
<213> Artificial Sequence

<220>
<223> Description of Artificial Sequence: Synthetic
      peptide

<400> 50
Met Asn Arg Arg Gly Ser Ile Lys Gln Ala Lys Ile
1               5                   10

<210> 51
<211> 22
<212> PRT
<213> Artificial Sequence

<220>
<223> Description of Artificial Sequence: Synthetic
      peptide

<400> 51
Met Phe Ala Val Arg Asp Arg Arg Gln Thr Val Lys Lys Gly Val Ile
1               5                   10                  15

Lys Ala Val Asp Ala Val

            20

<210> 52
<211> 14
<212> PRT
<213> Artificial Sequence

<220>
<223> Description of Artificial Sequence: Synthetic
      peptide

<400> 52
Phe Gly Glu Ser Arg Ala Ser Thr Phe Cys Gly Thr Pro Asp
1               5                   10

<210> 53
<211> 10
<212> PRT
<213> Artificial Sequence

<220>
<223> Description of Artificial Sequence: Synthetic
      peptide

<400> 53
Lys Ala Arg Leu Ser Tyr Ser Asp Lys Asn
1               5                   10

<210> 54
<211> 13
<212> PRT
<213> Artificial Sequence

<220>
<223> Description of Artificial Sequence: Synthetic
      peptide

<400> 54
Ser Ala Phe Ala Gly Phe Ser Phe Val Asn Pro Lys Phe
1               5                   10

<210> 55
<211> 22
<212> PRT
<213> Artificial Sequence

<220>
<223> Description of Artificial Sequence: Synthetic
      peptide

<400> 55
Lys Lys Lys Lys Lys Arg Phe Ser Phe Lys Lys Ser Phe Lys Leu Ser
1               5                   10                  15

Gly Phe Ser Phe Lys Lys
            20

<210> 56
<211> 20
<212> DNA
<213> Artificial Sequence

<220>
<223> Description of Artificial Sequence: Synthetic
      oligonucleotide

<400> 56
gttctcgctg gtgagtttca                                                    20


<210> 57
<211> 21
<212> PRT
<213> Artificial Sequence

<220>
<223> Description of Artificial Sequence: Synthetic
      peptide

<400> 57
Gly Ile Val Glu Gln Cys Cys Thr Ser Ile Cys Ser Leu Tyr Gln Leu
1               5                   10                  15


Glu Asn Tyr Cys Asn
            20


<210> 58
<211> 30
<212> PRT
<213> Artificial Sequence

<220>
<223> Description of Artificial Sequence: Synthetic
      peptide

<400> 58
Phe Val Asn Gln His Leu Cys Gly Ser His Leu Val Glu Ala Leu Tyr
1               5                   10                  15


Leu Val Cys Gly Glu Arg Gly Phe Phe Tyr Thr Lys Pro Thr
            20                  25                  30


<210> 59
<211> 30
<212> PRT
<213> Artificial Sequence

<220>
<223> Description of Artificial Sequence: Synthetic
      peptide

<400> 59

Phe Val Asn Gln His Leu Cys Gly Ser His Leu Val Glu Ala Leu Tyr
1               5                   10                  15

Leu Val Cys Gly Glu Arg Gly Phe Phe Tyr Thr Asp Lys Thr
            20                  25                  30

<210> 60
<211> 21
<212> PRT
<213> Artificial Sequence

<220>
<223> Description of Artificial Sequence: Synthetic
      peptide

<400> 60
Gly Ile Val Glu Gln Cys Cys Thr Ser Ile Cys Ser Leu Tyr Gln Leu
1               5                   10                  15

Glu Asn Tyr Cys Gly
            20

<210> 61
<211> 32
<212> PRT
<213> Artificial Sequence

<220>
<223> Description of Artificial Sequence: Synthetic
      peptide

<400> 61
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

<210> 62
<211> 30
<212> PRT
<213> Artificial Sequence

<220>
<223> Description of Artificial Sequence: Synthetic
      peptide

<400> 62
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr
            20                  25                  30

<210> 63
<211> 337
<212> PRT
<213> Homo sapiens

<400> 63
Pro Pro Asn Thr Ser Lys Val Tyr Ser Tyr Phe Glu Cys Arg Glu Lys
1               5                   10                  15

Lys Thr Glu Asn Ser Lys Leu Arg Lys Val Lys Tyr Glu Glu Thr Val
            20                  25                  30

Phe Tyr Gly Leu Gln Tyr Ile Leu Asn Lys Tyr Leu Lys Gly Lys Val
        35                  40                  45

Val Thr Lys Glu Lys Ile Gln Glu Ala Lys Asp Val Tyr Lys Glu His
    50                  55                  60

Phe Gln Asp Asp Val Phe Asn Glu Lys Gly Trp Asn Tyr Ile Leu Glu
65                  70                  75                  80

Lys Tyr Asp Gly His Leu Pro Ile Glu Ile Lys Ala Val Pro Glu Gly
                85                  90                  95

Phe Val Ile Pro Arg Gly Asn Val Leu Phe Thr Val Glu Asn Thr Asp
            100                 105                 110

Pro Glu Cys Tyr Trp Leu Thr Asn Trp Ile Glu Thr Ile Leu Val Gln
        115                 120                 125

Ser Trp Tyr Pro Ile Thr Val Ala Thr Asn Ser Arg Glu Gln Lys Lys
    130                 135                 140

Ile Leu Ala Lys Tyr Leu Leu Glu Thr Ser Gly Asn Leu Asp Gly Leu
145                 150                 155                 160

Glu Tyr Lys Leu His Asp Phe Gly Tyr Arg Gly Val Ser Ser Gln Glu
                165                 170                 175

Thr Ala Gly Ile Gly Ala Ser Ala His Leu Val Asn Phe Lys Gly Thr
            180                 185                 190

Asp Thr Val Ala Gly Leu Ala Leu Ile Lys Lys Tyr Tyr Gly Thr Lys
        195                 200                 205

Asp Pro Val Pro Gly Tyr Ser Val Pro Ala Ala Glu His Ser Thr Ile
    210                 215                 220

Thr Ala Trp Gly Lys Asp His Glu Lys Asp Ala Phe Glu His Ile Val
225                 230                 235                 240

Thr Gln Phe Ser Ser Val Pro Val Ser Val Val Ser Asp Ser Tyr Asp
                245                 250                 255

Ile Tyr Asn Ala Cys Glu Lys Ile Trp Gly Glu Asp Leu Arg His Leu
            260                 265                 270

Ile Val Ser Arg Ser Thr Gln Ala Pro Leu Ile Ile Arg Pro Asp Ser
        275                 280                 285

Gly Asn Pro Leu Asp Thr Val Leu Lys Val Leu Glu Ile Leu Gly Lys
    290                 295                 300

Lys Phe Pro Val Thr Glu Asn Ser Lys Gly Tyr Lys Leu Leu Pro Pro
305                 310                 315                 320

Tyr Leu Arg Val Ile Gln Gly Asp Gly Val Asp Ile Asn Thr Leu Gln
                325                 330                 335

Glu

<210> 64
<211> 491
<212> PRT
<213> Mus sp.

<400> 64
Met Asn Ala Ala Ala Glu Ala Glu Phe Asn Ile Leu Leu Ala Thr Asp
  1               5                  10                  15

Ser Tyr Lys Val Thr His Tyr Lys Gln Tyr Pro Pro Asn Thr Ser Lys
             20                  25                  30

Val Tyr Ser Tyr Phe Glu Cys Arg Glu Lys Lys Thr Glu Asn Ser Lys
         35                  40                  45

Val Arg Lys Val Lys Tyr Glu Glu Thr Val Phe Tyr Gly Leu Gln Tyr
     50                  55                  60

Ile Leu Asn Lys Tyr Leu Lys Gly Lys Val Val Thr Lys Glu Lys Ile
 65                  70                  75                  80

Gln Glu Ala Lys Glu Val Tyr Arg Glu His Phe Gln Asp Asp Val Phe
                 85                  90                  95

Asn Glu Arg Gly Trp Asn Tyr Ile Leu Glu Lys Tyr Asp Gly His Leu
            100                 105                 110

Pro Ile Glu Val Lys Ala Val Pro Glu Gly Ser Val Ile Pro Arg Gly
        115                 120                 125

Asn Val Leu Phe Thr Val Glu Asn Thr Asp Pro Glu Cys Tyr Trp Leu
    130                 135                 140

Thr Asn Trp Ile Glu Thr Ile Leu Val Gln Ser Trp Tyr Pro Ile Thr
145                 150                 155                 160

Val Ala Thr Asn Ser Arg Glu Gln Lys Lys Ile Leu Ala Lys Tyr Leu
                165                 170                 175

Leu Glu Thr Ser Gly Asn Leu Asp Gly Leu Glu Tyr Lys Leu His Asp
            180                 185                 190

Phe Gly Tyr Arg Gly Val Ser Ser Gln Glu Thr Ala Gly Ile Gly Ala
        195                 200                 205

Ser Ala His Leu Val Asn Phe Lys Gly Thr Asp Thr Val Ala Gly Ile
    210                 215                 220

Ala Leu Ile Lys Lys Tyr Tyr Gly Thr Lys Asp Pro Val Pro Gly Tyr

225                 230                 235                 240

Ser Val Pro Ala Ala Glu His Ser Thr Ile Thr Ala Trp Gly Lys Asp
                245                 250                 255

His Glu Lys Asp Ala Phe Glu His Ile Val Thr Gln Phe Ser Ser Val
            260                 265                 270

Pro Val Ser Val Val Ser Asp Ser Tyr Asp Ile Tyr Asn Ala Cys Glu
        275                 280                 285

Lys Ile Trp Gly Glu Asp Leu Arg His Leu Ile Val Ser Arg Ser Thr
    290                 295                 300

Glu Ala Pro Leu Ile Ile Arg Pro Asp Ser Gly Asn Pro Leu Asp Thr
305                 310                 315                 320

Val Leu Lys Val Leu Asp Ile Leu Gly Lys Lys Phe Pro Val Thr Glu
                325                 330                 335

Asn Ser Lys Gly Tyr Lys Leu Leu Pro Pro Tyr Leu Arg Val Ile Gln
            340                 345                 350

Gly Asp Gly Val Asp Ile Asn Thr Leu Gln Glu Ile Val Glu Gly Met
        355                 360                 365

Lys Gln Lys Lys Trp Ser Ile Glu Asn Val Ser Phe Gly Ser Gly Gly
    370                 375                 380

Ala Leu Leu Gln Lys Leu Thr Arg Asp Leu Leu Asn Cys Ser Phe Lys
385                 390                 395                 400

Cys Ser Tyr Val Val Thr Asn Gly Leu Gly Val Asn Val Phe Lys Asp
                405                 410                 415

Pro Val Ala Asp Pro Asn Lys Arg Ser Lys Lys Gly Arg Leu Ser Leu
            420                 425                 430

His Arg Thr Pro Ala Gly Asn Phe Val Thr Leu Glu Glu Gly Lys Gly
        435                 440                 445

Asp Leu Glu Glu Tyr Gly His Asp Leu Leu His Thr Val Phe Lys Asn
    450                 455                 460

Gly Lys Val Thr Lys Ser Tyr Ser Phe Asp Glu Val Arg Lys Asn Ala
465                 470                 475                 480

Gln Leu Asn Ile Glu Gln Asp Val Ala Pro His
                485                 490